# EUROPEAN PATENT APPLICATION

(11) **EP 4 818 297 A1**
(43) Date of publication of application: **07.10.2026**
(21) Application number: 26167889.0
(22) Date of filing: 26.03.2026
(51) Int. Cl.: C11D 3/18, C11D 3/382, C11D 3/50, C11D 3/20, A61K 8/92, C11D 1/62, C11D 3/00

(54) **TREATMENT COMPOSITIONS CONTAINING PARTICLES HAVING CERTAIN RESINS**

(30) Priority: 04.04.2025 EP 25168605
(71) Applicant: The Procter & Gamble Company, Cincinnati, OH 45202 (US)
(72) Inventor: BAGLIONI, Michele, 50019 Sesto Fiorentino (IT); BAGLIONI, Piero, 50019 Sesto Fiorentino (IT); CAMERINI, Rachel Elisabetta, 50019 Sestio Fiorentino (IT); COLLU, Mattia, 1853 Strombeek-Bever (BE); ORLANDINI, Laura, 1213 Petit-Lancy - Geneva (CH); SMETS, Johan, 1853 Strombeek-Bever (BE); TAHON, Cedric Marc, 1853 Strombeek-Bever (BE); TONELLI, Monica, 50019 Sesto Fiorentino (IT)
(74) Representative: P&G Patent Belgium UK

(57) **Abstract**

The present invention relates to treatment compositions comprising particles and an adjunct ingredient, wherein the particles comprise a resin and one or more benefit agents, wherein the one or more benefit agents comprises a fragrance material. Said resin is selected from a rosin ester, a terpene polymer resin, or mixtures thereof. The rosin ester is made from an at least one plant rosin acid linked to an alcohol by at least one ester bond, wherein the alcohol is selected from a linear sugar alcohol, a glycol, a triol or a mixture thereof. The terpene polymer resin is an alpha-pinene polymer.

## Description

### FIELD OF THE INVENTION

The present disclosure relates to slurries and treatment compositions that include particles, where the particles include certain resins and one or more benefit agents. The present disclosure also relates to methods of making and using such compositions.

### BACKGROUND OF THE INVENTION

Treatment compositions, such as liquid fabric softeners, can employ various delivery systems to facilitate improved delivery of benefit agents. Particles are commonly selected as a delivery system, particularly for perfumes.

Resins, such as rosin esters, may function as a perfume delivery agent when incorporated into particles. As rosin esters are derived from natural and/or sustainable sources, they constitute an alternative to synthetic materials, such as synthetic polymers.

Due to their intrinsic stickiness, rosin esters provide an enhanced deposition efficiency and high resistance to water dilution which result in improved fabric odor and customer delight across all touchpoints.

However, rosin esters in the finished product may cause undesirable residues on the walls of the container. Consumers may associate this phenomenon with a loss of product or with an unstable and/or spoiled product.

There is an ongoing need for treatment compositions that include resin-based delivery systems with improved stability, dispersibility and processability.

It is an object of the present invention to provide a resin-based liquid treatment composition that does not leave residues on the walls of the container, i.e. is homogeneous and stable over time, while maintaining the deposition benefits offered by the resins.

It is another object of the present invention to provide a method of making said treatment composition.

It is another object of the present invention to provide a method of treating fabric comprising using said treatment composition.

### SUMMARY OF THE INVENTION

It was surprisingly found that particles made with particular resins reduce the stickiness of resin-based treatment compositions to the walls of the container compared to particles made from different resin materials, without compromising on the benefit agent deposition efficiency.

In a first aspect, the present invention relates to a treatment composition comprising particles and an adjunct ingredient, wherein the particles comprise a resin and one or more benefit agents, wherein the one or more benefit agents comprises a fragrance material, characterized in that the resin is selected from a rosin ester, a terpene polymer resin, or mixtures thereof, wherein the rosin ester has the following formula: 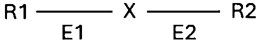 wherein R1 and R2 are plant rosin acids or H, wherein at least one of R1 or R2 is a plant rosin acid, wherein E1 and E2 are covalent bonds, wherein at least one of E1 or E2 is an ester bond, wherein X is an alcohol selected from the following: a) a sugar alcohol selected from _erythritol, pentaerythritol, xylitol, arabitol, ribitol, or mixtures thereof, b) a glycol comprising between 2 and 5 repeating subunits, wherein the repeating subunits are selected from an ethylene glycol, a 1,2-propylene glycol, a 1,3-propylene glycol, or mixtures thereof, c) a triol selected from glycerol, butanetriol, pentantriol, esantriol or mixtures thereof, or d) mixtures thereof, wherein X has two terminal oxygen atoms, wherein at least one of the oxygen atoms is configured to engage with at least one of the ester bonds, and wherein the rosin ester comprises rosin diesters and at least one second material, wherein the second material comprises at least one component, wherein, if: i) the second material comprises only one component, then the concentration in the composition of rosin diesters is higher than said one component, or ii) the second material comprises two or more components, then the concentration in the composition of rosin diesters is higher than any single component of the second material, wherein the terpene polymer resin is an alpha-pinene polymer having between 3 and 5 α-pinene monomers.

In a second aspect, the present invention relates to a method of making the treatment composition according to the first aspect, said process comprising the following steps: A) mixing a resin and one or more benefit agents to create an encapsulated perfume slurry; and B) using the slurry to produce the treatment composition.

In a third aspect, the present invention also relates to a method of treating fabrics, the method comprising the following steps: A) obtaining a fabric to be washed; and B) contacting the fabric with a wash liquor, the wash liquor comprising the treatment composition to the first aspect and water.

In a fourth aspect, the present invention also relates to an encapsulated perfume slurry.

### DETAILED DESCRIPTION

The present invention relates to slurries and treatment compositions comprising resins and benefit agents, such as fragrances, as part of the same delivery particle. The resins are selected from rosin esters, terpene polymer resin, or mixtures thereof.

The components, compositions, and processes of the present disclosure are described in more detail below.

As used herein, the articles "a" and "an" when used in a claim, are understood to mean one or more of what is claimed or described. As used herein, the terms "include," "includes," and "including" are meant to be non-limiting. The compositions of the present disclosure can comprise, consist essentially of, or consist of, the components of the present disclosure.

The terms "substantially free of" or "substantially free from" may be used herein. This means that the indicated material is at the very minimum not deliberately added to the composition to form part of it, or, preferably, is not present at analytically detectable levels. It is meant to include compositions whereby the indicated material is present only as an impurity in one of the other materials deliberately included. The indicated material may be present, if at all, at a level of less than 1%, or less than 0.1%, or less than 0.01%, or even 0%, by weight of the composition.

The slurries of the present invention, i.e. encapsulated perfume slurries, may be used to produce the treatment compositions describe hereafter by the method of making described below in the description.

The treatment composition may be a liquid treatment composition or a solid treatment composition, preferably a liquid treatment composition. More preferably, the treatment composition is a liquid fabric care treatment composition, a liquid hard surface treatment composition, a liquid personal care treatment composition, most preferably a liquid fabric care treatment composition.

The compositions of the present disclosure may be selected from the group of light duty liquid detergents compositions, heavy duty liquid detergent compositions, detergent gels commonly used for laundry, bleaching compositions, laundry additives, fabric enhancer compositions, hard surface cleaners and mixtures thereof.

The composition may be in any suitable form. The composition may be in the form of a liquid composition, a granular composition, a single-compartment pouch, a multi-compartment pouch, a sheet, a pastille or bead, a fibrous article, a tablet, a bar, flake, or a mixture thereof. The composition can be selected from a liquid, solid, or combination thereof.

The composition can be an aqueous liquid laundry detergent composition. For such aqueous liquid laundry detergent compositions, the water content can be present at a level of from 5.0 %to 95 %, preferably from 25 %to 90 %, more preferably from 50 % to 85 % by weight of the liquid detergent composition.

The pH range of the detergent composition is from 6.0 to 8.9, preferably from pH 7 to 8.8.

The detergent composition can also be encapsulated in a water-soluble film, to form a unit dose article. Such unit dose articles comprise a detergent composition of the present invention, wherein the detergent composition comprises less than 20%, preferably less than 15%, more preferably less than 10% by weight of water, and the detergent composition is enclosed in a water-soluble or dispersible film. Such unit-dose articles can be formed using any means known in the art. Suitable unit-dose articles can comprise one compartment, wherein the compartment comprises the liquid laundry detergent composition. Alternatively, the unit-dose articles can be multi-compartment unit-dose articles, wherein at least one compartment comprises the liquid laundry detergent composition.

The treatment composition may be a solid treatment composition. The skilled person is aware of the composition and properties of solid treatment compositions. The solid treatment composition may be a granular composition comprising a plurality of granules. The granules may be spray dried, agglomerated, extruded or a mixture thereof. Typically, the granular laundry detergent composition is a fully formulated laundry detergent composition, not a portion thereof such as a spray-dried, extruded or agglomerate granule that only forms part of the laundry detergent composition.

The laundry treatment composition may be in the form of a water-soluble porous flexible solid sheet. The water-soluble porous flexible solid sheet may comprise a water-soluble polymer, a surfactant.

The laundry treatment composition may be in the form of a fabric enhancer bead. Said fabric enhancer beads may comprise a water-soluble carrier. The water-soluble carrier acts to carry the delivery particle to the wash liquor. Upon dissolution of the water-soluble carrier, the delivery particle is dispersed into the wash liquor and deposited onto the laundry.

The water-soluble carrier can be a material that is soluble in a wash liquor within a short period of time, for instance less than about 10 minutes.

The laundry treatment composition may be in the form of a solid dissolvable composition. Solid dissolvable compositions (SDC) comprise crystallizing agent, water, and an active agent. The crystallizing agent primarily consists of sodium salt of saturated fatty acids having from 8 to 12 methylene groups, where the sodium counter ion enables formation of fiber crystal mesh (counter ions other than sodium result in non-fiber crystals) in the composition.

The treatment composition may be a liquid hard surface treatment composition. The skilled person is aware of the composition and properties of liquid hard surface treatment compositions.

The liquid hard surface composition can be an aqueous cleaning composition. The cleaning composition can comprise more than 50%, preferably more than 60% by weight of the composition of water. The composition may comprise from 80% to 99.5%, optionally from 85% to 99%, optionally from 94% to 98%, optionally from 95% to 99.93%, optionally from 97% to 99.93%, optionally from 98% to 99.9%, optionally from 98% to 99.5%, optionally from 98% to 99%, water by weight of the cleaning composition.

The cleaning composition can comprise from 0.05% to 4% perfume by weight of the cleaning composition. Perfume includes aromatic compounds, including but not limited to, essential oils and synthetic fragrance ingredients and mixtures thereof.

The cleaning composition can comprise 0.02% to 10%, optionally from 0.04% to 5%, optionally from 0.1% to 4%, optionally from about 0.5% to about 2%, and any ranges within any of the aforesaid ranges to two decimal places, nonionic surfactant by weight of the cleaning composition. The nonionic surfactant can be an ethoxylated non-ionic surfactant, an alkyl polyglucoside, and mixtures thereof.

The cleaning composition may comprise up to 5% by weight, optionally up to 3%, optionally up to 1%, of an additional surfactant, preferably selected from: anionic, amphoteric, zwitterionic, and mixtures thereof. The zwitterionic surfactant may comprise an amine oxide.

The cleaning composition can comprise a solvent system comprising C₁-C₄ alcohols, glycol ether solvents, and mixtures thereof.

The cleaning composition can have a pH from about 2 to about 11, optionally from about 2 to about 5, optionally from about 6 to about 9, optionally from about 8 to about 11.

The treatment composition may be a liquid personal care treatment composition. The skilled person is aware of the composition and properties of liquid personal care treatment compositions. The liquid personal care composition may comprise greater than about 1% by weight of a surfactant system which provides cleaning performance to the composition or may comprise greater than 5% by weight of a surfactant system which enables solubilization of a scalp care active. In addition, the composition may have sufficient surfactant to enable micellar or polymeric thickening. The surfactant system comprises an anionic surfactant and/or a combination of anionic surfactants and/or a combination of anionic surfactants and co-surfactants selected from the group consisting of amphoteric, zwitterionic, nonionic and mixtures thereof.

The personal care composition may comprise from about 10% to about 23%, from about 12% to about 21%, from about 10% to about 18%, by weight of one or more surfactants.

Anionic surfactants suitable for use in the compositions are the alkyl, alkyl ether sulfates, anionic alkyl and alkyl ether sulfosuccinates and/or dialkyl and dialkyl ether sulfosuccinates and mixtures thereof. The personal care composition may comprise a co-surfactant. The co-surfactant can be selected from the group consisting of amphoteric surfactant, zwitterionic surfactant, non-ionic surfactant and mixtures thereof. The co-surfactant can include, but is not limited to, lauramidopropyl betaine, cocoamidopropyl betaine, lauryl hydroxysultaine, sodium lauroamphoacetate, disodium cocoamphodiacetate, cocamide monoethanolamide, alkyl glycosides, alkyl polyglucosides, glyceril ester, polyglyceryl esters, sorbitan esters, alkylphenol ethoxylates, and mixtures thereof. The personal care composition may further comprise from about 0.5% to about 8%, from about 1.0% to about 7%, from about 1.5% to about 6%, by weight of one or more amphoteric, zwitterionic, nonionic co-surfactants, or a mixture thereof.

The present composition may comprise a humectant. The composition contains a safe and effective amount of the humectant. In particular, it may contain from about 20% to about 70%; from about 20% to about 50%; from about 23% to about 45%; by weight of a humectant. The composition may contain two or more different humectants, for example, the composition may contain glycerin and xylitol.

The personal care composition may comprise a thickening polymer to increase the viscosity of the composition. Suitable thickening polymers can be used. The personal care composition may comprise from about 0.05% to about 10% of a thickening polymer, from about 0.05% to about 5% of a thickening polymer, from about 0.05%to about 2.5%% of a thickening polymer, and from about 0.05%% to about 2% of a thickening polymer. The thickening polymer modifier may be a polyacrylate, polyacrylamide thickeners. The thickening polymer may be an anionic thickening polymer. The thickening polymers may be combined with cellulose and derivatives, with a guar and guar derivatives, with polyethylene oxide:polypropylene oxide; and POE-PPO copolymers.

The present composition may comprise a scalp care active. The scalp care active includes soluble scalp care actives and scalp health agents.

The treatment composition may be a conditioning composition, such as a liquid fabric enhancer composition or a hair conditioner composition.

Most preferably, the treatment composition may be a liquid fabric care treatment composition. The liquid fabric care treatment composition can be Newtonian or non-Newtonian, preferably non-Newtonian. The liquid fabric care treatment composition can comprise from 5% to 99%, or from 15% to 90%, or from 25% to 80% by weight of the liquid fabric care composition of water.

As used herein the phrase "fabric care composition" includes compositions and formulations designed for treating fabric. Such compositions include but are not limited to, laundry cleaning compositions and detergents, fabric softening compositions, fabric enhancing compositions, fabric freshening compositions, laundry prewash, laundry pretreat, laundry additives, spray products, dry cleaning agent or composition, laundry rinse additive, wash additive, post-rinse fabric treatment, ironing aid, unit dose formulation, delayed delivery formulation, detergent contained on or in a porous substrate or nonwoven sheet, and other suitable forms that may be apparent to one skilled in the art in view of the teachings herein. Such compositions may be used as a pre-laundering treatment, a post-laundering treatment, or may be added during the rinse or wash cycle of the laundering operation.

Unless otherwise noted, all component or composition levels are in reference to the active portion of that component or composition, and are exclusive of impurities, for example, residual solvents or by-products, which may be present in commercially available sources of such components or compositions.

All temperatures herein are in degrees Celsius (°C) unless otherwise indicated. Unless otherwise specified, all measurements herein are conducted at 20°C and under the atmospheric pressure.

In all aspects of the present disclosure, all percentages are by weight of the total composition, unless specifically stated otherwise. All ratios are weight ratios, unless specifically stated otherwise.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

### Treatment composition

The present disclosure relates to liquid treatment compositions that comprise particles and an adjunct ingredient.

The treatment compositions of the present disclosure may be fabric care treatment compositions. Such compositions may be used as a pre-laundering treatment, a post-laundering treatment, or may be added during the rinse or wash cycle of the laundering operation. The fabric care composition may be a fabric detergent composition, a fabric conditioning composition, or a mixture thereof, preferably a fabric conditioning composition. Fabric conditioning compositions may include liquid fabric softeners and liquid fabric enhancing compositions.

The treatment composition may be encapsulated in a water-soluble film, so as to be in the form of a unitized unit dose article, such as a pouch. The water-soluble film may be a polyvinyl alcohol water-soluble film. Suitable films are available from MonoSol, LLC (Indiana, USA).

The treatment composition can be encapsulated in a single or multi-compartment pouch. A multi-compartment pouch may have at least two, at least three, or at least four compartments. A multi-compartmented pouch may include compartments that are side-by-side and/or superposed. The composition contained in the pouch or compartments thereof may be liquid, solid (such as powders), or combinations thereof; in such cases, at least one encapsulated composition is a liquid composition.

The treatment composition may be in the form of a unit dose article, such as a pouch or a sachet, the composition may be encapsulated by a water-soluble film. A water-soluble unit dose article may comprise at least one water-soluble film shaped such that the unit dose article comprises at least one internal compartment surrounded by the water-soluble film.

The at least one compartment comprises the treatment composition. The unit dose article may comprise more than one compartment, even at least two compartments, or even at least three compartments, or even at least four compartments, or even at least five compartments. The compartments may be arranged in superposed orientation, i.e. one positioned on top of the other. Alternatively, the compartments may be positioned in a side-by-side orientation, i.e. one orientated next to the other. The compartments may even be orientated in a "tire and rim" arrangement, i.e. a first compartment is positioned next to a second compartment, but the first compartment at least partially surrounds the second compartment but does not completely enclose the second compartment. Alternatively, one compartment may be completely enclosed within another compartment. When one compartment comprises a liquid composition according to the present disclosure, another compartment may comprise a solid, a liquid, or a mixture thereof.

The film of the present invention may be soluble or dispersible in water (e.g., at 20°C). Preferred film materials include polymeric materials. The film material can, for example, be obtained by casting, blow-moulding, extrusion or blown extrusion of the polymeric material, as known in the art. Preferably, the water-soluble film comprises polyvinyl alcohol polymer or copolymer, preferably a blend of polyvinylalcohol polymers and/or polyvinylalcohol copolymers, preferably selected from sulphonated and carboxylated anionic polyvinylalcohol copolymers especially carboxylated anionic polyvinylalcohol copolymers, most preferably a blend of a polyvinylalcohol homopolymer and a carboxylated anionic polyvinylalcohol copolymer. Suitable films include those supplied by MonoSol, LLC (Indiana) under the trade references M8630, M8900, M8779, and/or M8310. The film may comprise an aversive agent, for example a bittering agent. Prior to be being formed into a unit dose article, the water-soluble film preferably has a thickness of from 20 to 150 microns, preferably 35 to 125 microns, even more preferably 50 to 110 microns, most preferably 76 microns.

The treatment composition may be in the form of a liquid. The liquid composition may include from 30%, or from 40%, or from 50%, to 99%, or to 95%, or to 90%, or to 75%, or to 70%, or to 60%, by weight of the composition, of water. The liquid composition may be a liquid laundry detergent, a liquid fabric conditioner, a liquid dish detergent, a hair shampoo, a hair conditioner, or a mixture thereof.

The composition may be characterized by a viscosity. The composition may have a viscosity of from 1 to 1500 centipoises (1-1500 mPa*s), from 50 to 5 1000 centipoises (50-1000 mPa*s), or from 100 to 500 centipoises (100-500 mPa*s), or from 100 to 200 centipoises (100-200 mPa*s), at 20 s-1 and 21°C, is disclosed. Relatively lower viscosities allow for improved dosing and/or less residue in a dispenser drawer. Viscosity is determined according to the method provided in the Test Methods section below.

The treatment compositions of the present disclosure may be characterized by a pH of from 2 to 12, or from 2 to 8.5, or from 2 to 7, or from 2 to 5. The treatment compositions of the present disclosure may have a pH of from 2 to 4, preferably a pH of from 2 to 3.7, more preferably a pH from 2 to 3.5, preferably in the form of an aqueous liquid. It is believed that such pH levels facilitate the stability of certain adjuncts, such as conditioning actives (e.g., esterquats). The pH of a composition is determined by dissolving/dispersing the composition in deionized water to form a solution at 10% concentration, at 20°C.

In a first aspect, the invention relates to a treatment composition comprising particles and an adjunct ingredient, wherein the particles comprise a resin and one or more benefit agents, wherein the one or more benefit agents comprises a fragrance material, characterized in that the resin is selected from a rosin ester, a terpene polymer resin, or mixtures thereof, wherein the rosin ester has the following formula: 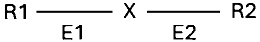
wherein R1 and R2 are plant rosin acids or H, wherein at least one of R1 or R2 is a plant rosin acid,
wherein E1 and E2 are covalent bonds, wherein at least one of E1 or E2 is an ester bond,
wherein X is an alcohol selected from the following:
   a) a sugar alcohol selected from _erythritol, pentaerythritol, xylitol, arabitol, ribitol, or mixtures thereof,
   b) a glycol comprising between 2 and 5 repeating subunits, wherein the repeating subunits are selected from an ethylene glycol, a 1,2-propylene glycol, a 1,3-propylene glycol, or mixtures thereof,
   c) a triol selected from glycerol, butanetriol, pentantriol, esantriol or mixtures thereof, or
   d) mixtures thereof,
wherein X has two terminal oxygen atoms, wherein at least one of the oxygen atoms is configured to engage with at least one of the ester bonds, and wherein the rosin ester comprises rosin diesters and at least one second material, wherein the second material comprises at least one component, wherein, if: i) the second material comprises only one component, then the concentration in the composition of rosin diesters is higher than said one component, or ii) the second material comprises two or more components, then the concentration in the composition of rosin diesters is higher than any single component of the second material, wherein the terpene polymer resin is an alpha-pinene polymer having between 3 and 5 α-pinene monomers.

Alternatively, the invention relates to a treatment composition comprising particles and an adjunct ingredient, wherein the particles comprise a resin and one or more benefit agents, wherein the one or more benefit agents comprises a fragrance material, characterized in that the resin is a rosin ester, wherein the rosin ester has the following formula: 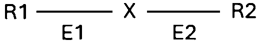
wherein R1 and R2 are plant rosin acids or H, wherein at least one of R1 or R2 is a plant rosin acid,
wherein E1 and E2 are covalent bonds, wherein at least one of E1 or E2 is an ester bond,
wherein X is an alcohol selected from the following:
   a) a sugar alcohol selected from _erythritol, pentaerythritol, xylitol, arabitol, ribitol, or mixtures thereof,
   b) a glycol comprising between 2 and 5 repeating subunits, wherein the repeating subunits are selected from an ethylene glycol, a 1,2-propylene glycol, a 1,3-propylene glycol, or mixtures thereof,
   c) a triol selected from glycerol, butanetriol, pentantriol, esantriol or mixtures thereof, or
   d) mixtures thereof,
   wherein X has two terminal oxygen atoms, wherein at least one of the oxygen atoms is configured to engage with at least one of the ester bonds, and wherein the rosin ester comprises rosin diesters and at least one second material, wherein the second material comprises at least one component, wherein, if: i) the second material comprises only one component, then the concentration in the composition of rosin diesters is higher than said one component, or ii) the second material comprises two or more components, then the concentration in the composition of rosin diesters is higher than any single component of the second material.

The second material comprises one or more components, preferably a plurality of components. The components may be rosin derivatives, rosin monoesters, free (unesterified) plant rosin acids, (free) alcohols, or mixtures thereof. Preferably, the components may be rosin monoesters, free (unesterified) plant rosin acids, (free) alcohols, or mixtures thereof. Each individual component of the second material has a concentration, i.e. % by weight of the composition lower than the concentration of the rosin diesters. The rosin diesters may have a concentration that is higher, equal or lower than the concentration of the second material, i.e. of the sum of all the components.

If the second material comprises only one component, then the concentration in the composition of rosin diesters is higher than said one component. If the second material comprises two or more components, then the concentration in the composition of rosin diesters is higher than any single component, i.e. any individual component, of the second material. In the latter case, the sum of the components, i.e. the second material, may have a higher concentration of the rosin diesters, while each single component of the second material has a lower concentration than the rosin diesters.

Alternatively, the first aspect may relate to a fabric care treatment composition, preferably a liquid fabric care treatment composition.

Alternatively, the treatment composition may comprise particles and an adjunct ingredient, wherein the particles comprise a resin and one or more benefit agents, wherein the one or more benefit agents comprises a fragrance material, characterized in that the resin is selected from a rosin ester, a terpene polymer resin, or mixtures thereof, wherein, the rosin ester is selected from an abietic-type acid, a derivative of an abietic-type acid, or a mixture thereof, and the rosin ester is derived from a plant rosin acid and an alcohol, wherein the alcohol comprises a middle chain and two terminal hydroxyl groups, wherein the middle chain comprises between 2 and 5 repeating subunits, wherein each of the repeating subunits comprises at least one oxygen atom and between 2 and 3 carbon atoms, and the rosin ester comprises rosin diesters and at least one second material, wherein the plant rosin diesters are more present than the at least one second material in the rosin ester, wherein the terpene polymer resin is an alpha-pinene polymer.

When the resin and the benefit agents, i.e. fragrances, are mixed together, they form encapsulates and/or particles spontaneously. The properties of these particles are described later in the description. The composition also comprises an adjunct ingredient which will also be described later in the description. Rosin esters have high resistance to dilution and optimal deposition on fabrics which make them optimal delivery agents for fragrances. However, treatment compositions containing rosin esters often have an increased stickiness towards the material of commonly-used containers, thus leaving undesirable and non-resuspendable residues and/or pellets. Surprisingly, resins according to the present invention, reduce the buildup of residues on the walls of containers.

Without wishing to be bound by theory, the use of these resins allows a greater degree of emulsification and oil-water phase stability, thus preventing the formation of sticky residues on the walls of the container. It is believed that the rosin esters according to the present invention may present an hydrophilic core being represented by the unesterified part of the alcohol which can improve the emulsification properties of the rosin esters.

The containers and their internal walls may be made from, but are not limited to, plastic (e.g. PET), glass or combinations thereof.

Furthermore, resins according to the present invention lead to a stabilization of the particles. The stability of the particles may be assessed by optical microscopy according to the Test Method described below.

Without wishing to be bound by theory, the use of these resins leads to the formation of particles as part of a stable emulsion.

Additionally, it was surprisingly found that the treatment compositions retained the same level of particle deposition, i.e. fragrance deposition, onto the fabric. It is believed that the intrinsic stickiness of resins towards the fabric is maintained, whereas it is selectively diminished against the container's material.

Upon fabric treatment, the deposited resin, in the form of particles, releases the fragrance throughout washing/drying steps and during fabric use by the consumer. It is believed that the consumer might want to remove completely the deposited resin in the following laundry to avoid resin build up in the form of macroscopic resin stains. The removal of these stains may be difficult to achieve, even using high-active detergents, because of the strong association between the resin and the fabric.

It was surprisingly found that, resin deposited on fabrics by treatment compositions according to the present invention are more easily cleanable compared to the same fabrics stained by treatment compositions having different types of resin. It is believed that the use of these specific resins, allows for an easier removal of the deposited resin when the fabric is washed with water and suitable detergents.

The skilled is aware of suitable detergents that would be able to remove deposited resin from fabrics. Surfactant systems as described below may be suitable for this purpose.

Suitable rosin esters according to the present invention may be Dertoline DEG2 and Granolite TEG.

### Delivery Particles (or "Particles")

The present disclosure relates to particles, which are intended as "delivery particles". The particles comprise a resin and one or more benefit agent. The particles may be hard capsules, soft capsules or mixtures thereof. The particles may form a suspension or an emulsion in the water environment. When the particles are hard capsules, a suspension is formed. A suspension is considered a heterogeneous mixture in which solid particles are dispersed throughout a liquid or gas but are not dissolved. When the particles are soft capsules, an emulsion is formed. An emulsion is considered a type of colloidal mixture in which two immiscible liquids, typically oil and water, are combined with the help of an emulsifying agent. The composition may preferably be characterized by an emulsion of soft capsules inside a water environment.

Without wishing to be bound by theory, the benefit agent alone would form immiscible and unstable droplets into the water environment. When solid rosin esters are heated and mixed with the benefit agents, soft capsules form, where the resin functions as an emulsifying agent, thus creating a stable emulsion inside the water environment.

The one or more benefit agents may be encapsulated in the resin, and/or embedded in the resin. Compositions of the present disclosure may comprise the presently described particles.

The particles of the present disclosure may be present in a population, which may have a volume-weighted average diameter (or "diameter" as used herein). Volume-weighted average diameter is determined according to the method provided in the Test Method section below.

The particles may be characterized by a volume-weighted median particle size between more than 0 um and 400 um, or between more than 0 um and 300 um, or between more than 0 um and 250 um, or between more than 0 um and 200 um, or between 0 um and 150 um, or between 0 um and 125 um, preferably between more than 0 um and 100 um, more preferably between more than 0 um and 90 um.

In a preferred aspect of the present invention, the particles may be characterized by a volume-weighted median particle size between 0.5 um and 40 um, preferably between 1 um and 20 um, more preferably between 1 um and 10 um, wherein the volume-weighted median particle size is calculated according to the Test methods described below.

We surprisingly found that the particles according to the present invention were generally smaller and more stable compared to a composition with different types of resins. It is believed that the resins are able to stabilize relatively small particles, wherein the particles' size corresponds to an optimal compromise between good particle deposition properties and reduced stickiness to the container's material.

One or more particles of the present disclosure may comprise at least one region comprising a benefit agent, such as a perfume raw material or enzyme. The region may comprise a benefit agent, such as fragrance, being encompassed or encapsulated within the resin.

One or more particles of the present disclosure may have a structure selected from the group consisting of: (a) a particle comprising a single region having benefit agent that is embedded in the resin; (b) a particle comprising at least two regions having benefit agents that are embedded in the resin; (c) a particle comprising at least one region having benefit agents that is at least partially embedded on the surface of the resin; (d) a particle comprising a single region having a benefit agent that is embedded in the resin and at least one region having a benefit agent that is at least partially embedded on the surface of the resin; and (e) a particle comprising at least two regions having benefit agents that are embedded in the resin and at least one region having a benefit agent that is at least partially embedded on the surface of the resin. Compositions of the present disclosure may include one or more particles having a structure according to (a)-(e), or mixtures thereof.

The particles may be characterized by a weight ratio of the resin to the benefit agent (e.g., fragrance). In a preferred aspect of the present invention, the weight ratio between the resin and the fragrance material in the composition may be 1:4 to 4:1, preferably 1:4 to 2:1, more preferably 1:3 to 1.5:1, most preferably 1:1. Weight ratios of resin to benefit agent closer to 1:1 may be preferred to deliver the primary benefit, i.e. reduced stickiness to the walls of the container;

The compositions of the present disclosure may comprise between 0.01% and 10%, or between 0.05% and 7%, or between 0.1% and 5%, more preferably between 0.8 % and 4 %, or between 1% and 3%, by weight of the composition, of the particles.

The benefit agents of the particles are described in more detail below.

### Resins

The composition of the present invention comprises resins. A resin describes a solid or a viscous substance that is typically organic in nature. It is believed that the resins will interact with the benefit agents, as disclosed herein, to form an effective perfume delivery system. In particular, resins provide performance benefits, for example by facilitating improved deposition and/or stability of benefit agents.

Resins may be present from 0.01% to 10%, or from 0.01% to 5%, or from 0.05% to 3%, or from 0.1% to 1%, by weight of the treatment composition. Adding too little may result in little to no added benefit, while adding too much may result in processing challenges.

Resins may be characterized by a softening point. Resins are typically solid at room temperature, but the softening point is a measure of the glass transition temperature associated with these materials. The softening point of a resin is determined according to method provided in the Test Method section below. The resin, e.g rosin esters, may be characterized by a softening point of from 0°C to 175°C, or from 0°C to 150°C, or from 0°C to 125°C. If the softening point of the resin is equal or lower to room temperature, the resin may be partially or completely in liquid form. Resins may need to be softened by heating in order to be incorporated into consumer products. Thus, for ease of processing and/or energy savings, resin having relatively low softening points may be preferred for the compositions and processes of the present disclosure. Low softening points may also have an effect on improving the deposition aid performance of the resin.

Resins, e.g rosin esters, may be characterized by an acid number (sometimes called "acid value"). The acid number of a resin relates to the total free acid content of these products. The acid number of a resin is determined according to method provided in the Test Method section below.

Resins, e.g rosin esters, may be characterized by an acid number less than 175, e.g., from 0 to 175. For the particles, compositions, and processes of the present disclosure, it may be preferred to use rosin esters having a relatively low acid number, such as less than 125, preferably less than 100, more preferably less than 75, even more preferably less than 50, more preferably less than 25, so as to have minimal effect on the final pH of the treatment composition.

The color of the resin, e.g a rosin ester, may be graded based on the Gardner Color standard number, ranging 1 to 18. So as to have minimal effect on the final color of the treatment composition, preferred resins of the present disclosure may have a color grade of from 1 to 10, preferably from 1 to 8. The color grade of a resin is determined according to method provided in the Test Method section below.

Resins are typically relatively insoluble in water. For example, resins according to the present disclosure may be characterized by a solubility of less than 1 g/L, or less than 100g/L, or less than 1g/L, or less than 0.1g/L, or less than 0.01g/L, in deionized water at 22°C. Without wishing to be bound by theory, it is believed that the relatively insoluble nature of the resin of the present disclosure contribute to the deposition efficiency and performance of the associated benefit agent.

Resins may be characterized by a density. Typically, the resins are characterized by a density of at more than 1.0 kg/dm3, preferably at least 1.1 kg/dm3, at 25°C.

Resins are typically flammable. For the particles, compositions, and processes of the present disclosure, it may be preferred to use resins that have a relatively high flash point, e.g., higher than 190°C, to facilitate easier and safer processing. The flash point of a resin is determined according to method provided in the Test Method section below.

In a preferred aspect of the present invention, the resin may have a softening point of less than 75°C, preferably of less than 50°C, more preferably of less than 45°C, most preferably of between 35°C and 45°C.

In another preferred aspect of the present invention, the resin may be characterized by at least one of the two following characteristics: a) an acid value between more than 0 and 20, preferably between 13 and 20, or even between 13 and 15, b) a color grade between 1 and 10, preferably between 1 and 7, more preferably between 2 and 6 as graded on the Gardner Color standard number.

Without wishing to be bound by theory, a low softening point (below 75°C) and appropriate acid value or Gardner color, confers to the resin preferrable characteristics improving the technical benefit described above. The skilled person knows how to determine the softening point, the acid value and the Gardner color of a resin. These properties are described more in detail below.

The resin is selected from rosin ester, a terpene polymer resin, or mixtures thereof.

The rosin ester has the following formula: 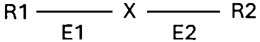
wherein R1 and R2 are plant rosin acids or H, wherein at least one of R1 or R2 is a plant rosin acid,
wherein E1 and E2 are covalent bonds, wherein at least one of E1 or E2 is an ester bond,
wherein X is an alcohol selected from the following:
   a) a sugar alcohol selected from _erythritol, pentaerythritol, xylitol, arabitol, ribitol, or mixtures thereof,
   b) a glycol comprising between 2 and 5 repeating subunits, wherein the repeating subunits are selected from an ethylene glycol, a 1,2-propylene glycol, a 1,3-propylene glycol, or mixtures thereof,
   c) a triol selected from glycerol, butanetriol, pentantriol, esantriol or mixtures thereof, or
   d) mixtures thereof,
wherein X has two terminal oxygen atoms, wherein at least one of the oxygen atoms is configured to engage with at least one of the ester bonds.

The rosin ester is an ester of at least one plant rosin acid and an alcohol. It is believed that, the rosin esters are formed by esterification of at least one terminal hydroxyl group of the alcohol with at least one plant rosin acid to form rosin monoesters and rosin diesters. The rosin ester may comprise rosin diesters, rosin monoesters, plant rosin acids, alcohol or mixtures thereof.

In a preferred aspect of the present invention, the at least one plant rosin acid may be an abietic acid, and the at least one plant rosin acid may be selected from a gum plant rosin acid, a wood plant rosin acid, a tall oil plant rosin acid, preferably a tall oil plant rosin acid. The plant rosin acids will be described more in detail below.

The alcohol comprises a middle chain and two terminal hydroxyl groups.

In another aspect, X may be an alcohol selected from the following: a sugar alcohol selected from_erythritol, pentaerythritol, xylitol, arabitol, ribitol, or mixtures thereof.

In another aspect, X may be an alcohol selected from the following: a linear sugar alcohol selected from _erythritol, xylitol, arabitol, ribitol, or mixtures thereof.

In another aspect, X may be an alcohol selected from the following: a glycol comprising between 2 and 5 repeating subunits, wherein the repeating subunits are selected from an ethylene glycol, a 1,2-propylene glycol, a 1,3-propylene glycol, or mixtures thereof.

In another aspect, X may be an alcohol selected from the following: a triol selected from glycerol, butanetriol, pentantriol, esantriol or mixtures thereof,

In another aspect, X may be an alcohol selected from the following:
- a sugar alcohol selected from_erythritol, pentaerythritol, xylitol, arabitol, ribitol, or mixtures thereof,
- a glycol comprising between 2 and 5 repeating subunits, wherein the repeating subunits are selected from an ethylene glycol, a 1,2-propylene glycol, a 1,3-propylene glycol, or mixtures thereof,
   or
- mixtures thereof.

In another aspect, X may be an alcohol selected from the following:
- a sugar alcohol selected from_erythritol, pentaerythritol, xylitol, arabitol, ribitol, or mixtures thereof,
- a triol selected from glycerol, butanetriol, pentantriol, esantriol or mixtures thereof, or
- mixtures thereof.

In another aspect, X may be an alcohol selected from the following:
- a glycol comprising between 2 and 5 repeating subunits, wherein the repeating subunits are selected from an ethylene glycol, a 1,2-propylene glycol, a 1,3-propylene glycol, or mixtures thereof,
- a triol selected from glycerol, butanetriol, pentantriol, esantriol or mixtures thereof, or
- mixtures thereof.

The alcohol may be a sugar alcohol. The sugar alcohol may be a linear sugar alcohol. The skilled person is aware of the structure of sugar alcohols.

The alcohol may be a triol. The triol may be selected from glycerol, butanetriol, pentantriol, esantriol or mixtures thereof. Alternatively, the triol may be selected from butanetriol, pentantriol, esantriol or mixtures thereof.

A suitable butanetriol may be a 1,2,4 butanetriol.

A suitable pentantriol may be a 1,2,5-pentantriol, a 1,3,5-pentantriol, or mixtures thereof.

A suitable esantriol may be a 1,2,6-esantriol, a 1,3,6-esantriol, or mixtures thereof.

In another aspect, X may be an alcohol selected from the following:
- a sugar alcohol selected from_erythritol, pentaerythritol, xylitol, arabitol, ribitol, or mixtures thereof,
- a triol selected from butanetriol, pentantriol, esantriol or mixtures thereof, or
- mixtures thereof.

In another aspect, X may be an alcohol selected from the following:
- a glycol comprising between 2 and 5 repeating subunits, wherein the repeating subunits are selected from an ethylene glycol, a 1,2-propylene glycol, a 1,3-propylene glycol, or mixtures thereof,
- a triol selected from butanetriol, pentantriol, esantriol or mixtures thereof, or
- mixtures thereof.

The alcohol may comprise ether groups in the middle chain. The alcohol may be a glycol. The glycol may comprise between 2 and 5 repeating subunits, wherein the repeating subunits may be selected from an ethylene glycol, a 1,2-propylene glycol, a 1,3-propylene glycol, or mixtures thereof,

Suitable alcohols may be selected from di-, tri-, poly- ethylene glycols, or di-, tri-, poly-1,2-propylene glycols, or di-, tri-, poly- 1,3-propylene glycols, or mixtures thereof. The term poly-indicates up to five repeating subunits.

It is to be intended, and it is clear to the skilled person, that said classes of glycols may be combined as part of the same molecule, or as different molecules in a mixture of molecules, or combinations thereof, to form the alcohol.

The middle chain may be linear or branched. The middle chain of the alcohol is preferably linear. It is believed that, the absence of branching in the alcohol, reduces the sterical hindrance, thus improving the stability of the resin derived from said alcohol.

Preferred suitable alcohols may be selected from mono-, di-, tri-, poly- ethylene glycols, or di-, tri-, poly- 1-3-propylene glycols, or combinations thereof.

In another preferred aspect of the present invention, the alcohol may be selected from diethylene glycol, triethylene glycol, or mixtures thereof, preferably diethylene glycol.

The rosin ester comprises rosin diesters and at least one second material, wherein the rosin diesters are more present than each of the at least one second material in the rosin ester.

The second material may be selected from a rosin monoester, a plant rosin acid, an alcohol or mixtures thereof. A plant rosin acid may be intended as a free plant rosin acid, i.e. non-esterified plant rosin acid. The presence of rosin monoesters and of plant rosin acids may be due to an incomplete esterification between the plant rosin acid and the alcohol. The second material may be present as a residual portion of the rosin ester. The second material may be substantially free of plant rosin acids.

The rosin diesters are produced by the esterification of two plant rosin acids with two hydroxyl groups of the alcohol.

In a preferred aspect of the present invention, the rosin diesters may constitute at least 45%, preferably at least 70%, more preferably at least 75%, by weight of the rosin ester.

In a preferred aspect of the present invention, the weight ratio between the rosin diesters and at least one second material may be 9:1 to 1:1.5, preferably 8:1 to 1:1, more preferably 4:1 to 2:1, most preferably 3:1, preferably, wherein the at least one component is selected from a rosin monoester, the at least one plant rosin acid, the alcohol or mixtures thereof.

It is believed that, the production of said rosin esters may result in different degrees of esterification leading to a mixture of rosin diesters, rosin monoesters, free plant rosin acids and free alcohol molecules, wherein the rosin diesters represent the major component in the mixture.

The at least one second material may include a certain amount of unreacted alcohol, preferably the at least one second material is substantially free from unreacted alcohol. Alternatively, the rosin esters may be selected from compounds of the following formulas: 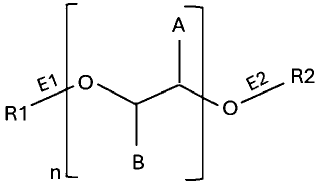
wherein R1 and R2 are plant rosin acids or H, wherein at least one of R1 or R2 is a plant rosin acid,
wherein E1 and E2 are covalent bonds, wherein at least one of E1 or E2 is an ester bond,
wherein n is 2 to 5, A is H or CH3, wherein B is H,
   or 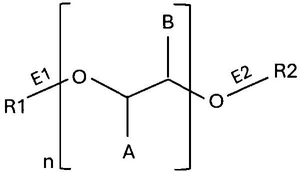
wherein R1 and R2 are plant rosin acids or H, wherein at least one of R1 or R2 is a plant rosin acid,
wherein E1 and E2 are covalent bonds, wherein at least one of E1 or E2 is an ester bond,
wherein n is 2 to 5, A is H or CH3, wherein B is H,
   or 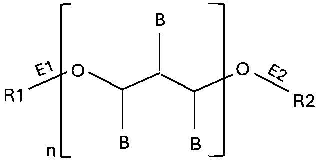
wherein R1 and R2 are plant rosin acids or H, wherein at least one of R1 or R2 is a plant rosin acid,
wherein E1 and E2 are covalent bonds, wherein at least one of E1 or E2 is an ester bond, wherein n is 2 to 5, wherein B is H,
   or
mixtures thereof.

Rosin diesters are characterized by both R1 and R2 being plant rosin acids and both E1 and E2 being ester bonds. Rosin monoesters are characterized by only one of R1 and R2 being a plant rosin acid and the corresponding bond (E1 or E2) being an ester bond.

The terpene polymer resin is an alpha-pinene polymer. The skilled person is aware of the chemical structure of terpene polymer resins and of alpha-pinene polymers.

Terpene monomers are a by-product in the extraction of resin from wood stumps or tree sap, and from the extraction of oils from citrus fruits. The latter is the dominant source. Terpenes polymer resins are cyclic, which is partly responsible for their excellent solvent properties.

Terpenes polymer resins according to the present invention are polymers resulting from the polymerization of α-pinene monomers, β-pinene monomers, or combinations thereof, preferably α-pinene monomers.
α-pinene polymers have the following formula. 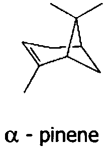
β-pinene monomers have the following formula:

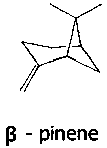

Terpenes polymer resins according to the present invention may be polymers resulting from the polymerization of between 2 and 20, preferably between 2 and 12, more preferably between 3 and 9, most preferably between 3 and 6, α-pinene monomers, β-pinene monomers, or combinations thereof, preferably α-pinene monomers.

In other words, the terpene polymer resin may be a polymer comprising between 2 and 20, preferably between 2 and 12, more preferably between 3 and 9, most preferably between 3 and 6, monomers selected from α-pinene monomers, β-pinene monomers, or combinations thereof, preferably α-pinene monomers.

In a preferred aspect of the present invention, the terpenes polymer resin is a polymer comprising between 3 and 6 monomers selected from α-pinene monomers, β-pinene monomers, or combinations thereof, preferably α-pinene monomers.

In another preferred aspect of the present invention, the terpenes polymer resin is a polymer comprising between 3 and 6 α-pinene monomers.

The terpene polymer resin may have a weight molecular weight between 250 g/mol and 3000 g/mol, preferably between 400 g/mol and 2000 g/mol, more preferably between 400 g/mol and 1100 g/mol, most preferably between 400 g/mol and 700 g/mol.

T_{g}, or glass transition temperature, is a critical thermal property of polymers that indicates the temperature at which a polymer transitions from a hard, glassy state to a more flexible, rubbery state.

The terpene polymer resin may preferably have a Tg of less than 0 °C, preferably between -10 °C and -30 °C.

The skilled person is aware of suitable polymers of α-pinene monomers and β-pinene monomers. One non-limiting example of suitable commercially available terpene polymer is Dercolyte LTG (DRT).

### Rosin esters

The resins of the present disclosure may be rosin esters. As defined herein, a rosin ester is a derivative of a plant rosin acid. The rosin ester is one type of rosin acid derivative. Rosin esters may further be modified.

Plant rosin acids may be modified to form rosin acid derivatives. Plant rosin acids may be selected from plant rosin acids, rosin acid derivatives or mixtures thereof.

Plant rosin acids are naturally-derived, i.e. extracted from plants. Plant rosin acids are monocarboxylic acids with a diterpenic structure. Diterpenes are a class of terpenes composed of four isoprene units, often with the molecular formula C20H32. Non-limited examples of plant rosin acids are shown in Table A. Plant rosin acids are further described below.

In one aspect, the weight ratio between the rosin ester and the fragrance material in the composition may be 1:4 to 4:1, preferably 1:4 to 2:1, more preferably 1:3 to 1.5:1, most preferably 1:1. Plant rosin acids and rosin esters are discussed in more detail below.

### a) Plant rosin acids

Plant rosin acids are typically derived from conifer plants (class: Pinopsida), usually from pine trees (genus: Pinus). Plant rosin acids are obtained from the plant's oleo-resin, which is distilled to remove volatile terpenes, and the solid material left behind are the plant rosin acids. The solid plant rosin acids may be relatively translucent and/or glass-like.

Plant rosin acids are monocarboxylic acids having three fused rings. Plant rosin acids may be tricyclic diterpene monocarboxylic acids, for example with a molecular formula of C19H29COOH.

A plant rosin acid may be an abietic acid, a pimaric acid, a plicatic acid, or a mixture thereof. Abietic acid may comprise different isomers such as abietic acid, neoabietic acid, dehydroabietic acid, palustric acid, levopimaric acid, or a mixture thereof. Pimaric acid may comprise different isomers such as pimaric acid, isopimaric acid, sandaracopimaric acid, or mixtures thereof. Structures of these illustrative plant rosin acids are provided below in Table A.

**Table A.**

| | | |
|---|---|---|
| 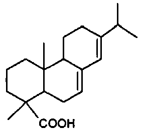 Abietic acid | 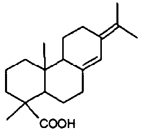 Neoabietic acid | 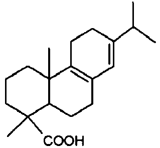 Palustric acid |
| 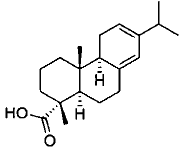 Levopimaric acid | 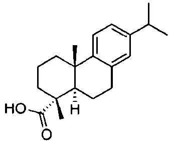 Dehvdroabietic acid | 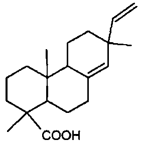 Pimaric acid |
| 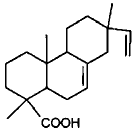 Isopimaric acid | 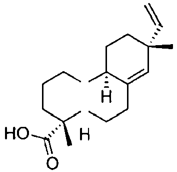 Sandaracopimiaric acid | 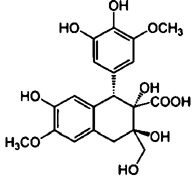 Plicatic acid |

Preferably, the plant rosin acid is an abietic acid. Abietic acid has the empirical formula C19H29COOH and is also known as abietinic acid or sylvic acid.

Plant rosin acids may be classified depending on the source where they are obtained. For example, plant rosin acids may be classified as gum plant rosin acid, wood plant rosin acid, tall oil plant rosin acid, or a mixture thereof. Gum plant rosin acid may be derived from a resin extrudate of a tree or other plant and may be harvested by tapping or wounding the tree and then collecting and processing the extrudate. Wood plant rosin acid may be derived from materials that are harvested from pine tree stumps, for example through solvent extraction and/or distillation. Tall oil plant rosin acid is a byproduct of the distillation of crude tall oil during the Kraft process of wood pulp manufacture when pulping pine trees.

Suitable plant rosin acids may be obtained, for example, from a variety of pine species, such as Pinus massoniana (Masson's pine), P. elliotti (slash pine), P. palustris (longleaf pine), P. taeda (loblolly pine), P. oocarpa (Mexican yellow pine), P. leiophylla (Chihuahua pine), P. devoniana (pino lacio, or Michoacan pine), P. montezumae (Montezuma pine), P. pinaster (maritime pine), P. sylvestris (Scots pine), P. halepensis (Aleppo pine), P. insularis (Benguet pine), P. kesiya (Khasi pine), P. strobus (Eastern white pine), or mixtures thereof.

The plant rosin acids may be selected from gum plant rosin acid, wood plant rosin acid, tall oil plant rosin acid, or mixtures thereof; preferably gum plant rosin acids.

In a preferred aspect, at least one plant rosin acid may be an abietic acid, and at least one plant rosin acid may be selected from a gum plant rosin acid, a wood plant rosin acid, a tall oil plant rosin acid, preferably a tall oil plant rosin acid.

### b) Rosin esters

Rosin esters are a type of rosin acid derivatives. Rosin esters may be made by chemically modifying a plant rosin acid, through an esterification reaction. The skilled person is aware of an esterification reaction between an acid and an alcohol. A rosin ester is the reaction product of a plant rosin acid and an alcohol, as exemplified below.

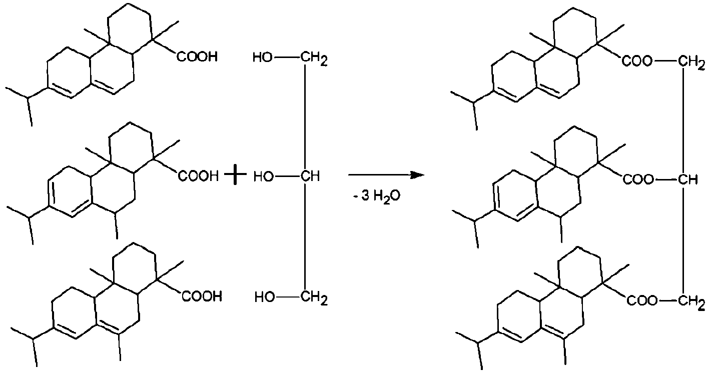

In the specific example shown, the rosin ester is formed by a condensation reaction (i.e. esterification) between three abietic acid molecules and one glycerol molecule. A rosin ester formed by an alcohol and one or more abietic acid molecules will be hereafter called abietic rosin ester.

The rosin ester may be the ester of an alcohol and of a plant rosin acid selected from an abietic acid, a pimaric acid, plicatic acid, or mixtures therof, preferably an abietic acid. It is therefore preferrable that the rosin ester is an abietic rosin ester.

The rosin ester may be the ester of an alcohol and of a plant rosin acid selected from a gum plant rosin acid, a wood plant rosin acid, a tall oil plant rosin acid, or mixtures thereof, preferably, a gum plant rosin acid. It is therefore preferrable that the rosin ester is a gum rosin ester.

The rosin ester may be an ester of a plant rosin acid and an alcohol, where the alcohol comprises: (a) from three to six carbon atoms, and/or (b) from two to six hydroxyl groups. The rosin ester may comprise, on average, from two to six moles of ester groups per mole of rosin ester.

Rosin esters may be esters of rosin acid derivatives and of an alcohol, wherein rosin acid derivatives are the result of synthetic modification of plant rosin acids from the processes of hydrogenation, dimerization, polymerization, saponification, or mixtures thereof.

The rosin acid derivative may be a hydrogenated rosin acid. Many plant rosin acids are unsaturated, they tend to be oxidatively unstable and may undergo color changes upon storage. Hydrogenation can help to stabilize the plant rosin acids and reduce undesirable color change. Furthermore, hydrogenated plant rosin acids tend to have lighter colors than the parent rosin, providing more formulation and aesthetic flexibility. Hydrogenated rosin acids may be partially or fully hydrogenated. Below is a sample reaction for the partial and full hydrogenation of abietic acid.

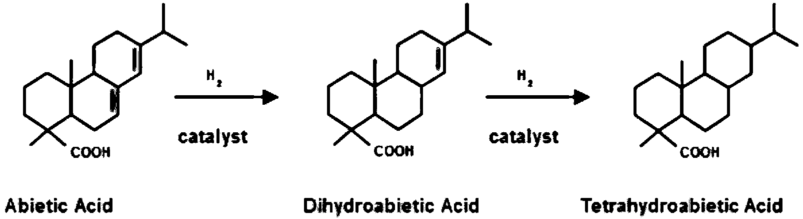

The rosin acid derivative may be a rosin acid dimer. Dimerization may be useful for increasing the softening point and/or stability of a plant rosin acid. A sample dimerization reaction of abietic acid is shown below.

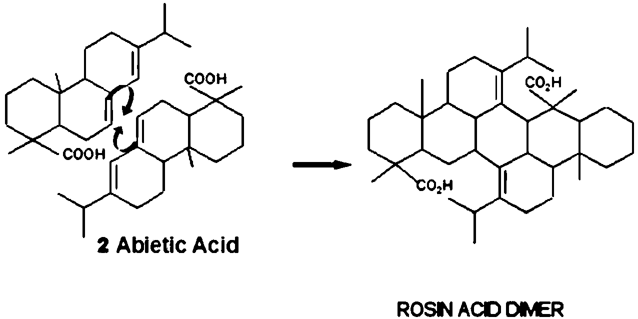

As it is difficult or even impossible to completely dimerize a sample of plant rosin acid, rosin acid dimers are often present with undimerized plant rosin acids. Rosin acid dimers may be further esterified.

A rosin acid derivative may be dimerized through ions such as Zi2+ or Ca2+. For example, zinc rosinates are rosin derivatives where two abietic acid compounds are bound to a zinc ion. The rosin acid derivative may be a rosin acid polymer. As used here, said polymer may comprise three or more monomeric units derived from plant rosin acids. The polymer may be a main-chain polymer or a side-chain polymer.

The rosin acid derivative may be a rosin acid soap, where a plant rosin acid is reacted with an alkali metal hydroxide (e.g., NaOH or KOH) or an alkaline earth metal hydroxide (e.g., Ca(OH)2). More broadly, the rosin derivative may be a rosin acid salt.

The rosin acid derivative may be a functionalized rosin acid. In other word, the rosin may be functionalized, where one or more functional groups are added to the plant rosin acid.

A rosin acid derivative may include the product of a Diels-Alder reaction, such as the reaction product of a plant rosin acid and maleic anhydride; such reaction products may be polymerized.

A rosin acid derivative may include phenolic rosin acids, where a plant rosin acid is reacted with a phenol.

A rosin acid derivative may include a rosin alcohol, wherein one or more of the carboxyl groups of the plant rosin acid are converted to hydroxyl groups.

Commercially available rosin acid derivatives that are suitable for the presently disclosed compositions and methods may include those disclosed in Example 1 of the Examples section below.

The plant rosin acid may be selected from plant rosin acids, rosin acid derivatives, or a mixture thereof.

Plant rosin acids may be selected from abietic acids, pimaric acids, plicatic acid, or mixtures thereof, preferably abietic acids.

Plant rosin acids may be selected from abietic acid, neoabietic acid, dehydroabietic acid, palustric acid, levopimaric acid, pimaric acid, isopimaric acid, sandaracopimaric acid, plicatic acid, gum plant rosin acids, wood plant rosin acids, tall oil plant rosin acids or mixtures thereof.

The rosin ester may be an ester of plant rosin acids, rosin acid dimers, rosin acid polymers, hydrogenated rosin acids, zinc rosinates, rosin acid soaps, rosin acid salts, functionalized rosin acids, phenolic rosin acids, rosin alcohol, or mixtures thereof, and of an alcohol.

The rosin ester may be an ester of abietic acid, neoabietic acid, dehydroabietic acid, palustric acid, levopimaric acid, pimaric acid, isopimaric acid, sandaracopimaric acid, plicatic acid, gum plant rosin acids, wood plant rosin acids, tall oil plant rosin acids, rosin acid dimers, rosin acid polymers, hydrogenated rosin acids, zinc rosinates, rosin acid soaps, rosin acid salts, functionalized rosin acids, phenolic rosin acids, rosin alcohol, or mixtures thereof, and of an alcohol.

### Benefit agents

The particles of the present disclosure comprise one or more benefit agents. As described above, it is believed that the benefit agents become embedded and/or encapsulated in resin when the particles are formed. The particle formation can thus lead to improved stability, delivery, and/or performance of the benefit agent on a target surface, such as a fabric or hard surface. For example, such embedding and/or encapsulation of a benefit agent may prevent degradation of the benefit agent and/or undesirable interactions with other components of the liquid consumer product.

The compositions of the present disclosure may include the benefit agent and/or particles containing the benefit at a level at which the benefit agent provides its intended benefit when the composition is used as intended. For example, the benefit agent of the particles may be present at a level of from 0.05% to 10%, or from 0.05% to 5%, or from 0.1% to 4%, by weight of the composition.

The benefit agent may be selected from the group consisting of fragrances, silicone oils, waxes, hydrocarbons, higher fatty acids, essential oils, lipids, skin coolants, vitamins, sunscreens, antioxidants, enzymes, malodor reducing agents, odor-controlling materials, antistatic agents, softening agents, insect and moth repelling agents, colorants, whiteness enhancers, defoamers, anti-foaming agents, UV protection agents for fabrics and skin, sun fade inhibitors, anti-allergenic agents, water proofing agents, skin care agents, glycerin, aloe vera, vitamin E, shea butter, cocoa butter, brighteners, antiperspirant actives, emollients, skin sensates, and mixtures thereof. Alternatively, the benefit agent may be selected from the group consisting of fragrances, waxes, hydrocarbons, essential oils, lipids, skin coolants, vitamins, sunscreens, antioxidants, malodor reducing agents, odor-controlling materials, antistatic agents, insect and moth repelling agents, colorants, whiteness enhancers, defoamers, anti-foaming agents, UV protection agents for fabrics and skin, sun fade inhibitors, anti-allergenic agents, water proofing agents, skin care agents, glycerin, aloe vera, vitamin E, shea butter, cocoa butter, antiperspirant actives, emollients, skin sensates, and mixtures thereof.

Particularly preferred benefit agents for the particles include fragrances. The delivery efficacy of the benefit agent may be most efficacious when the benefit agent is relatively hydrophobic. The benefit agent of the particles comprises fragrances, which may comprise one or more perfume raw materials. The term "perfume raw material" (or "PRM") as used herein refers to compounds having a molecular weight of at least 100 g/mol and which are useful in imparting an odor, fragrance, essence, or scent, either alone or with other perfume raw materials. Typical PRMs comprise inter alia alcohols, ketones, aldehydes, esters, ethers, nitrites, and alkenes, such as terpene. The skilled person is aware of suitable PRMs. Non-limiting examples of suitable PRMs is provided below.

Suitable PRMs may include materials such as geraniol, linalool, linalyl acetate, pyranol, geranyl acetate, anisaldehyde, citral, citronellal, lysmeral, citronellol, rose oxide, tetrahydrolinalool, hydroxycitronellal, betaionone, menthol, cinnamaldehyde, anethole, vanillin, ethyl vanillin, eugenol, cinnamon oil, carvone, piperonal, and mixtures thereof. The perfume raw materials may include naturally derived materials, such as essential oils.

The PRMs may be characterized by their boiling points (B.P.) measured at the normal pressure (760 mm Hg), and their octanol/water partitioning coefficient (P), which may be described in terms of logP, determined according to the test method below. Based on these characteristics, the PRMs may be categorized as Quadrant I, Quadrant II, Quadrant III, or Quadrant IV perfumes, as described in more detail below. A perfume having a variety of PRMs from different quadrants may be desirable, for example, to provide fragrance benefits at different touchpoints during normal usage.

The perfume raw materials may comprise a perfume raw material selected from the group consisting of perfume raw materials having a boiling point (B.P.) lower than 250°C and a LogP lower than 3, perfume raw materials having a B.P. of more than 250°C and a LogP of more than 3, perfume raw materials having a B.P. of more than 250°C and a LogP lower than 3, perfume raw materials having a B.P. lower than 250°C and a LogP more than 3 and mixtures thereof. Perfume raw materials having a boiling point B.P. lower than 250°C and a LogP lower than 3 are known as Quadrant I perfume raw materials. Quadrant 1 perfume raw materials are preferably limited to less than 30% of the fragrance. Perfume raw materials having a B.P. of more than 250°C and a LogP of more than 3 are known as Quadrant IV perfume raw materials, perfume raw materials having a B.P. of more than 250°C and a LogP lower than 3 are known as Quadrant II perfume raw materials, perfume raw materials having a B.P. lower than 250°C and a LogP more than 3 are known as a Quadrant III perfume raw materials.

The fragrance may comprise from 1% to 40%, by weight of the fragrance material, of Quadrant I perfume raw materials, and/or from 60% to 99%, by weight of the fragrance material, of non-Quadrant I perfume raw materials.

In particular, the delivery particles of the present disclosure are useful when the benefit agent of the particles contain hydrophobic perfume raw materials. The hydrophobic perfume raw materials may be characterized by a relatively high logP value, for example a logP of more than 3.0, and may include what is described above as Quadrant III PRMs, Quadrant IV PRMs, or mixtures thereof. The benefit agent of the particles may comprise at least 50%, or at least 60%, or at least 70%, or at least 80%, or at least 90%, or 100%, by weight of the benefit agent, of Quadrant III PRMs, Quadrant IV PRMs, or mixtures thereof. Compositions that comprise such levels of Quadrant III and/or IV PRMs as the benefit agent of the particles may be aqueous and comprise at least 60%, or at least 70%, or at least 80%, or at least 90%, or at least 95%, or at least 97%, by weight of the composition, of water, and/or less than 10%, or less than 5%, or less than 3%, surfactant.

Non-limiting examples of Quadrant III PRMs include iso-bomyl acetate, carvacrol, alphacitronellol, paracymene, dihydro myrcenol, geranyl acetate, d-limonene, linalyl acetate, vertenex, and mixtures thereof.

Non-limiting examples of Quadrant IV (or enduring) PRMs include allyl cyclohexane propionate, ambrettolide, amyl benzoate, amyl cinnamate, amyl cinnamic aldehyde, amyl cinnamic aldehyde dimethyl acetal, iso-amyl salicylate, hydroxycitronellal-methyl anthranilate (known as aurantiol^{®}), benzophenone, benzyl salicylate, para-tert-butyl cyclohexyl acetate, isobutyl quinoline, beta-caryophyllene, cadinene, cedrol, cedryl acetate, cedryl formate, cinnamyl cinnamate, cyclohexyl salicylate, cyclamen aldehyde, dihydro isojasmonate, diphenyl methane, diphenyl oxide, dodecalactone, 1-(1,2,3,4,5,6,7,8- octahydro-2,3,8,8-tetramethyl-2-naphthalenyl)-ethanone (known as iso E super^{®}), ethylene brassylate, methyl phenyl glycidate, ethyl undecylenate, 15-hydroxypentadecanoic acid lactone (known as exaltolide^{®}), 1,3,4,6,7,8-hexahydro- 4,6,6,7,8,8-hexamethyl-cyclopenta-gamma-2-benzopyran (known as galaxolide^{®}), geranyl anthranilate, geranyl phenyl acetate, hexadecanolide, hexenyl salicylate, hexyl cinnamic aldehyde, hexyl salicylate, alpha-irone, gamma-ionone, gamma-n-methyl ionone, para-tertiarybutyl- alpha-methyl hydrocinnamic aldehyde (known as lilial^{®}), lilial (p-t-bucinal)^{®}, linalyl benzoate, 2-methoxy naphthalene, methyl dihydrojasmone, musk indanone, musk ketone, musk tibetine, myristicin, oxahexadecanolide-10, oxahexadecanolide-11, patchouli alcohol, 5-acetyl- 1,1 ,2,3,3,6-hexamethylindan (known as phantolide^{®}), phenyl ethyl benzoate, phenylethylphenylacetate, phenyl heptanol, phenyl hexanol, alpha-santalol, delta-undecalactone, gamma-undecalactone, vetiveryl acetate, yara-yara, ylangene, and mixtures thereof.

In particular, the fragrance materials of the present disclosure may include perfume raw materials that are characterized by certain chemical or structural moieties.

In a preferred aspect of the present invention, the fragrance material may comprise one or more perfume raw material, wherein the one or more perfume raw material may comprise a moiety selected from the group consisting of a cycloalkane moiety, a cycloalkene moiety, a branched alkyl moiety, and combinations thereof, wherein the cycloalkane moiety may comprise a first ring structure having from five to seven carbons, preferably from five to six carbons, that form the first ring structure, wherein the cycloalkene moiety may comprise a second ring structure having from five to seven carbons, preferably from five to six carbons, that form the second ring structure, wherein the branched alkyl moiety comprises from one to four carbon atoms, preferably from two to three carbon atoms. Without wishing to be bound by theory, moieties of the indicated structures are likely to provide a better association with the resin compared to different moieties.

The one or more perfume raw materials may comprise at least two moieties selected from the group consisting of a cycloalkane moiety, a cycloalkene moiety, and a branched alkyl moiety.

The one or more perfume raw materials may be present between 25% and 100%, preferably between 50% and 95%, more preferably between 60% and 90%, even more preferably between 70% and 80%, by weight of the fragrance materials.

### Water

The liquid treatment compositions of the present disclosure may comprise water. The liquid treatment compositions according to the present disclosure may comprise between 1% and 99%, or between 10% and 99%, or between 10% and 96%, or between 12% and 90%, or between 20% and 80%, or between 40% and 80%, by weight of the composition, of water.

In particular, liquid compositions such as heavy-duty laundry detergents, liquid fabric conditioners (e.g., softeners or enhancers), and liquid hard surface cleaners may advantageously be formulated with high levels of water, for example to enhance flowability or dispersibility.

When the composition is in the form of a unit dose composition (for example, a liquid composition encapsulated by a water-soluble film), the water may be present at a level of between 1% and 20%, or between 5% and 15%, by weight of the composition. When the composition is in the form of a compact liquid laundry detergent, the water, may be present between 10% and 50%, or between 20% and 40%, by weight of the composition.

### Adjunct ingredient

The treatment compositions of the present disclosure may further include an adjunct ingredient in addition to the particles of the present disclosure. The adjuncts may be suitable for delivering a treatment benefit to a target surface, such as a fabric or other textile.

The adjunct ingredient(s) may be present in the composition at levels suitable for the intended use of the composition. Typical usage levels range from as low as 0.001% by weight of composition for adjuncts such as optical brighteners to 50% by weight of composition for builders.

The adjunct ingredient may be mixed with the slurry of the present invention to produce the treatment composition, especially when using the method of making described below.

The adjunct material is to be considered a structurally separate component of the treatment composition from the resin and/or the benefit agent. This means that the treatment composition comprises a resin and a benefit agent and an adjunct ingredient, where each of them is a separate component and together form the structure of the composition, despite the fact that they might have similar functional characteristics.

In a preferred aspect of the present invention, the adjunct ingredient may be selected from an amine, a surfactant system, a water binding agent, a sulfite, fatty acids and/or salts thereof, enzymes, encapsulated benefit agents, soil release polymers, hueing agents, builders, chelating agents, dye transfer inhibiting agents, dispersants, enzyme stabilizers, catalytic materials, bleaching agents, bleach catalysts, bleach activators, polymeric dispersing agents, soil removal/antiredeposition agents, polymeric dispersing agents, polymeric grease cleaning agents, brighteners, suds suppressors, dyes, hueing agents, free perfumes, encapsulated perfumes, a perfume delivery system, structure elasticizing agents, fabric softening agents, carriers, fillers, hydrotropes, organic solvents, anti-microbial agents and/or preservatives, neutralizers and/or pH adjusting agents, processing aids, fillers, rheology modifiers or structurants, opacifiers, pearlescent agents, pigments, anti-corrosion and/or anti-tarnishing agents, and mixtures thereof, preferably wherein the adjunct ingredient comprises a surfactant system, fabric softening agents or combinations thereof, more preferably wherein the surfactant system comprises anionic surfactant, non-ionic surfactant, cationic surfactant, and/or zwitterionic surfactant, and/or more preferably wherein the fabric softening agents comprise a quaternary ammonium compound, silicone compounds, or both, most preferably the adjunct ingredient is a quaternary ammonium compound.

Alternatively, the adjunct ingredient may be selected from an amine, a surfactant system, a water binding agent, a sulfite, soil release polymers, hueing agents, builders, chelating agents, dye transfer inhibiting agents, dispersants, enzyme stabilizers, catalytic materials, bleaching agents, bleach catalysts, bleach activators, polymeric dispersing agents, soil removal/antiredeposition agents, polymeric dispersing agents, polymeric grease cleaning agents, suds suppressors, dyes, hueing agents, structure elasticizing agents, fabric softening agents, carriers, fillers, hydrotropes, organic solvents, anti-microbial agents and/or preservatives, neutralizers and/or pH adjusting agents, processing aids, fillers, rheology modifiers or structurants, opacifiers, pearlescent agents, pigments, anti-corrosion and/or anti-tarnishing agents, and mixtures thereof, preferably wherein the adjunct ingredient comprises a surfactant system, fabric softening agents or combinations thereof, more preferably wherein the surfactant system comprises anionic surfactant, non-ionic surfactant, cationic surfactant, and/or zwitterionic surfactant, and/or more preferably wherein the fabric softening agents comprise a quaternary ammonium compound, silicone compounds, or both, most preferably the adjunct ingredient is a quaternary ammonium compound. Several of these adjuncts are described in more detail below.

The adjunct material may comprise a surfactant system, conditioning actives, or combinations thereof. Preferably, the surfactant system comprises anionic surfactant, nonionic surfactant, cationic surfactant, and/or zwitterionic surfactant. Preferably, the fabric softening agents comprise a quaternary ammonium compound, silicone compounds, or both.

Compositions according to the present disclosure may include a surfactant system. The surfactant system may include one or more surfactants.

The compositions of the present disclosure may include between 20% and 75%, or between 25% and 70%, or between 30% and 50%, by weight of the composition, of a surfactant system. Compositions of the present disclosure may include less than 20%, or less than 10%, or less than 5%, or less than 3%, by weight of the composition, of a surfactant system.

The surfactant system may include anionic surfactant, nonionic surfactant, zwitterionic surfactant, cationic surfactant, amphoteric surfactant, or combinations thereof. The surfactant system may include linear alkyl benzene sulfonate, alkyl ethoxylated sulfate, alkyl sulfate, nonionic surfactant such as ethoxylated alcohol, amine oxide, or mixtures thereof. The surfactants may be, at least in part, derived from natural sources, such as natural feedstock alcohols.

Suitable anionic surfactants may include any conventional anionic surfactant. This may include a sulfate detersive surfactant, for e.g., alkoxylated and/or non-alkoxylated alkyl sulfate materials, and/or sulfonic detersive surfactants, e.g., alkyl benzene sulfonates. The anionic surfactants may be linear, branched, or combinations thereof. Preferred surfactants include linear alkyl benzene sulfonate (LAS), alkyl ethoxylated sulfate (AES) including sodium laureth sulfate (SLES), alkyl sulfates (AS) including sodium lauryl sulfate (SLS), or mixtures thereof. Other suitable anionic surfactants include branched modified alkyl benzene sulfonates (MLAS), methyl ester sulfonates (MES), and/or alkyl ethoxylated carboxylates (AEC).

The surfactant system may include nonionic surfactant. Suitable nonionic surfactants include alkoxylated alkyl phenols, alkyl phenol condensates, mid-chain branched alcohols, mid-chain branched alkyl alkoxylates, alkylpolysaccharides (e.g., alkylpolyglycosides), polyhydroxy fatty acid amides, ether capped poly(oxyalkylated) alcohol surfactants, and mixtures thereof. The alkoxylate units may be ethyleneoxy units, propyleneoxy units, or mixtures thereof.

Suitable zwitterionic surfactants may include any conventional zwitterionic surfactant, such as betaines, including alkyl dimethyl betaine and cocodimethyl amidopropyl betaine, C8 to C18 (for example from C12 to C18) amine oxides (e.g., C12-14 dimethyl amine oxide), and/or sulfo and hydroxy betaines, such as N-alkyl-N,N-dimethylammino-1-propane sulfonate where the alkyl group can be C8 to C18, or from C10 to C14. The zwitterionic surfactant may include amine oxide.

The compositions of the present disclosure may include a fabric softening agent, also called conditioning active. Compositions that contain conditioning actives may provide softness, anti-wrinkle, anti-static, conditioning, anti-stretch, color, and/or appearance benefits. Conditioning actives suitable for compositions of the present disclosure may include quaternary ammonium ester compounds, silicones, non-ester quaternary ammonium compounds, amines, fatty esters, sucrose esters, silicones, dispersible triolefins, polysaccharides, fatty acids, softening or conditioning oils, polymer latexes, or combinations thereof. Preferably, the treatment composition comprises a conditioning active that comprises a quaternary ammonium ester compound, more preferably a quaternary ammonium ester compound in combination with a silicone. The composition may include between 1% and 40%, or between 1% and 30%, or between 5% and 30%, by weight of the composition, of conditioning active.

Liquid treatment compositions according to the present disclosure may include an external structurant. External structurants can provide physical stability to liquid compositions according to the present disclosure, for example by helping to suspend the delivery particles. Structurants, when present, are preferably present in an effective amount that is capable of suspending the particles in the treatment composition. External structurants may include non-polymeric crystalline, hydroxy-functional structurants and/or polymeric structurants.

Non-polymeric crystalline, hydroxyl functional structurants may comprise a crystallizable glyceride, which may be pre-emulsified to aid dispersion into the final detergent composition. Suitable crystallizable glycerides include hydrogenated castor oil or "HCO" or derivatives thereof, provided that it is capable of crystallizing in the liquid detergent composition. Polymeric structurants may include naturally derived structurants and/or synthetic structurants. Naturally derived polymeric structurants include: hydroxyethyl cellulose, hydrophobically modified hydroxyethyl cellulose, carboxymethyl cellulose, polysaccharide derivatives and mixtures thereof. Suitable polysaccharide derivatives include: pectine, alginate, arabinogalactan (gum Arabic), carrageenan, gellan gum, xanthan gum, guar gum and mixtures thereof. The structurant may comprise cellulosic fibers, for example in the form of microfibrillated cellulose. Cellulose may be derived from bacterial, wood, or other plants such as fruit or sugar beet.

Synthetic polymeric structurants include: polycarboxylates, polyacrylates, hydrophobically modified ethoxylated urethanes, hydrophobically modified non-ionic triols and mixtures thereof. The polycarboxylate polymer may be a polyacrylate, polymethacrylate or mixtures thereof. The polyacrylate may be a copolymer of unsaturated mono- or di-carbonic acid and C1-C30 alkyl ester of the (meth)acrylic acid. Such copolymers are available from Lubrizol Corp. under the tradename Carbopol^{®} Aqua 30.

Treatment compositions according to the present disclosure may include a further perfume delivery system. Suitable perfume delivery systems may include core-shell encapsulates, pro-perfumes (such as amine- and/or silicone-based pro-perfumes), and mixtures thereof. Core-shell encapsulates may comprise a core and a shell surrounding the core. The core may comprise a benefit agent such as perfume, and optionally a partitioning modifier such as isopropyl myristate. The shell may comprise an inorganic material, a polymer, for example melamine formaldehyde, polyurea, polyvinyl alcohol, polyacrylate, or a polysaccharide. Suitable encapsulates may be characterized by a volume-weighted median particle size of from 10 microns to 100 microns, or from 10 microns to 50 microns, or from 15 microns to 40 microns. Perfume delivery systems may provide benefits such as improved perfume stability, deposition, and/or longevity, and may be particularly useful for perfume raw materials that do not associate well with the resins of the present disclosure.

### Consumer product

In another aspect, the present invention relates to a consumer product comprising a bottle, wherein the treatment composition according to the first aspect is contained in the bottle.

The consumer product comprises a bottle. The bottle contains the treatment composition according to the present invention. The bottle may have a circular, oval or polygonal cross-section (e.g. rectangle, square, octagonal, etc.), or some combination of those cross-sections (e.g., a rectangular cross-section with outwardly bowed walls). The bottle, or at least a portion thereof, may be free of sharp angles to further reduce a risk of leakage.

The bottle may comprise a dimensionally stable shell constituting an external layer of the bottle. The dimensionally stable shell can be formed from compressed pulp.

The bottle may further comprise a layer of polymeric material coating an inner surface of the shell, the polymeric material may also be disposed on a portion of the exterior surface of the shell. However, the polymeric material may be limited to only the inner surface of the shell (i.e., the inside of bottle). The layer of polymeric material may delimit a cavity which is occupied at least in part by a liquid treatment composition.

The weight ratio of compressed pulp to polymeric material may be at least 50:50, more preferably at least 70:30, more preferably at least 80:20, more preferably at least 90:10, more preferably at least 95:5. In this context a weight ratio of "at least X:Y" should be understood as encompassing any value of ratio X1:Y1 where X1≥X and where Y1≤Y. These ratios are beneficial for the bottle to be processed according to various recycling streams. The polymeric material may comprise a triolefin or semi-crystalline polymeric material (see details below), which is applied to the inner surface of the shell as a fluid or powder.

The polymeric material can optimally fulfil the function of the barrier between the liquid conditioning compositions detailed herein and the fiber material and the environment. The shell and the layer of polymeric material therefore act as a composite two-layer material in which the individual layers, fulfill different tasks in order to obtain a fluid-tight and mechanically stable bottle.

The bottle may be closed with any closure known in the art, for example, a screw cap having an internal thread engaging an external thread of the neck of the bottle. Alternative closure can be provided to the bottle, such as snapped-in cap, rubber lid, cork, etc.

An external thread of the bottle can be coated by the polymeric material for providing additional mechanical stability and wear resistance to the screwing/unscrewing operations. Again, in other examples the polymeric material may be limited to only the inner surface of the shell (i.e., the inside of bottle.

The bottle may be provided with any shape and number of ribs for stability purposes. The ribs may be integrally formed with the fiber-based shell. The ribs may be located along the bottom of the bottle and/or sides of the bottle.

The polymeric material used for coating the inner surface of the shell is not limited. For example, the polymeric material may comprise a thermoplastic polymer selected from triolefins, e.g. polyethylene (incl. PET, LDPE or HDPE), or polypropylene and copolymers thereof, polyamides and polyesters, and copolymers thereof. The polymeric material may also comprise water soluble synthetic polymers, such as polyvinyl alcohol or polysaccharides, such as cellulose. The polymeric material may be coated from a powder, where the powder particles have an average size in the range of 1 to 200 µm, preferably 5 to 100 µm, more preferably 10 to 50 µm. These ranges allow for an even coating, and furthermore allow for charging of the polymeric powder particles to be accomplished by the spraying device.

In a further preferred aspect, the polymeric material may have a bio-based polymer (e.g., triolefin) content of at least 80% by weight, wherein the bio-base is defined according to the standards ASTM D 6866, CEN/TS 16137 and ISO 16620.

Conveniently, the bio-based polymer can be high density polyethylene (HDPE), low density polyethylene (LDPE), polyethylene terephthalate (PET), polyethylene furanoate (PEF), polyethylene isosorbide terephthalate (PEIT), polylactide (PLA), polybutylene succinate (PBS), poly-s-caprolactone (PCL) or polyhydroxyalkanoate (PHA), in particular polyhydroxybutyrate (PHB).

### Methods of making

A second aspect of the present invention relates to a method of making the treatment composition according to the first aspect, said process comprising the following steps: A) mixing a resin and one or more benefit agents to create an encapsulated perfume slurry; and B) using the slurry to produce the treatment composition, preferably, wherein the slurry is mixed with a cationic surfactant to produce the treatment composition, and wherein the benefit agent comprises a fragrance material, more preferably, wherein the cationic surfactant is a quaternary ammonium compound or a salt thereof.

It was surprisingly found that the process according to the present invention increase the processability of resin-based treatment compositions. The use of resins according to the present invention prevents the formation of insoluble residues when exposing stainless steel to the composition.

Without wishing to be bound by theory, the use of the resins improves the flowability of the composition in components made of stainless steel, potentially leading to processability benefits, e.g. during manufacturing and handling.

The present disclosure also relates to processes for making liquid treatment compositions.

The process of making a liquid treatment composition may comprise the step of combining the ingredients (e.g., resin, one or more benefit agents, a non-ionic surfactant and an adjunct material) as described herein.

The process of making a liquid treatment composition according to the present disclosure comprises the steps of combining the resin and the one or more benefit agents to form a slurry.

The slurry may comprise from 1% to 99%, by weight of the slurry, of the resin. The slurry may comprise from 1% to 99%, by weight of the slurry, of the benefit agent. The slurry may comprise the resin and the fragrance in a weight ratio of 1:4 to 4:1, preferably 1:4 to 2:1, more preferably 1:3 to 1.5:1, most preferably 1:1. It is believed that the stickiness benefit increases being closer to a weight ratio of 1:1.

If a solid resin is used, the particles forming therefrom may be hard capsules forming a suspension. If a liquid resin is used, the particles forming therefrom may be soft capsules forming an emulsion. Emulsions may be preferred over suspensions. Solid resins may be treated with agitation and heating to change their physical status to liquid, thus facilitating their processing and the formation of stable emulsions of the particles.

The resin may be heated to a temperature equal to or more than the softening point of the resin. The slurry may be made by combining the heated resin with the benefit agent, and mixing. In order to favor the homogeneity of the slurry, the mixing may take place in a heated oil bath set at a temperature equal to the softening point of the resin. As the samples become homogenous, the temperature can be progressively reduced, which helps to lower the risk of loss of volatile materials (e.g., evaporation of volatile PRMs). As an additional or alternative step to heating, the resin may be grinded to small particles and mixed with the benefit agent.

To make the slurry composition, the resin may be provided as small particles to facilitate homogenization of the slurry. If the resin is provided as solids that are larger than desired, the process may include grinding or pulverizing the resin into smaller particles. In a bench top operation, this may be accomplished with a mortar and pestle.

The slurry may comprise an emulsifying agent. The slurry may comprise from 1% to 95 %, or from 5% to 95%, preferably from 5 % to 40 % by weight of the slurry, of the emulsifying agent. The slurry may comprise the resin and the emulsifying agent in a weight ratio of from 5:95 to 95:5. The slurry may comprise the benefit agent and the emulsifying agent in a weight ratio of from 5:95 to 95:5. Suitable emulsifying agents may include surfactants, amphiphilic polymers, or mixtures thereof. The slurry may be substantially free (<5%) from, or even essentially free (<1%) from, emulsifying agents.

Suitable surfactants may include nonionic surfactants, anionic surfactants, or mixtures thereof.

Suitable amphiphilic polymers may include graft copolymers, such as poly(ethylene glycol)-poly(vinyl acetate) graft copolymer, polyvinyl caprolactam-polyvinyl acetate-polyethylene glycol graft copolymer, or mixtures thereof. Commercially available graft copolymers may include Sokalon^{®} HP 22 or Soluplus^{®}, both available from BASF.

In a preferred aspect of the present invention, the resin is grinded into pellets. The resin may be mixed with the fragrance to produce the slurry. The mixing may be performed by stirring with a magnetic bar. The mixture of the slurry composition and the base composition may need to be mixed at relatively high shear to ensure suitable incorporation / homogenization of the slurry into the base composition.

The resin may be provided at room temperature (e.g., 20-22°C). The mixture of plant resin and the one or more benefit agents may be heated prior to mixing, which can improve ease of homogenization. The mixture may be heated to a temperature up to the softening point of the plant resin, preferably to less than the softening point of the plant resin, more preferably to a temperature of from 20°C to 60°C, more preferably to a temperature of from 20°C to 30°C. A temperature of from 20°C to 30°C might be sufficient for mixing with the fragrance for producing a fully homogenized slurry. Without wishing to be bound by theory, the resin according to the present invention, having a relatively low softening point permit a rapid homogenization of the slurry even at relatively low temperatures.

It is preferred that the temperature is not too high, so as to avoid evaporation of volatile benefit agents, particularly fragrance material. Once made, the slurry may be stored at ambient temperatures.

The mixing at relatively elevated temperature may occur for variable period of time, from 30 seconds to 4 hours, or even from 30 seconds to 2 hours, or even from 30 seconds to 1 hours, or even from 30 seconds to 30 minutes, or even from 30 seconds to 10 minutes, or even from 30 seconds to 5 minutes, or even from 30 seconds to 1 minute. We surprisingly observed the homogenization of the slurry after mixing the ingredients by vortexing for 30 seconds to 1 minute. After the mixture has been substantially homogenized, the mixture may be cooled to ambient temperature. If the viscosity of the slurry at ambient temperature is lower than 6000cP, then the heating step may not be needed.

According to a preferred aspect of the present invention, the slurry may be mixed with a cationic surfactant to produce the treatment composition, preferably, wherein the cationic surfactant is a quaternary ammonium compound or a salt thereof.

The slurry may be added to an adjunct ingredient to produce the treatment composition according to the first aspect. Preferably, the adjunct ingredient comprises a fabric softening active or a conditioning active, preferably a quaternary ammonium compound.

The treatment composition may be formed by mixing the slurry and the adjunct ingredient by vortexing. Other adjunct materials may be added before, during, or after the addition of the slurry composition.

When using the slurry to make a final product composition, the slurry may be heated, for example heated to around 60°C or less, before being injected in the finished product or otherwise combined with a base composition. This heating step is most likely to be helpful when the slurry is characterized by a relatively high resin:benefit agent (e.g. fragrance) weight ratio, such as 1:1. The heating step may not be required.

Without wishing to be bound by theory, this order of addition improves the ease of homogenization of the mixture.

### Slurry

In another aspect, the invention relates to an encapsulated perfume slurry comprising particles, wherein the particles comprise a resin and one or more benefit agents, wherein the one or more benefit agents comprises a fragrance material, characterized in that the resin is selected from a rosin ester, a terpene polymer resin, or mixtures thereof, wherein the rosin ester has the following formula: 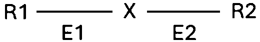 wherein R1 and R2 are plant rosin acids or H, wherein at least one of R1 or R2 is a plant rosin acid, wherein E1 and E2 are covalent bonds, wherein at least one of E1 or E2 is an ester bond, wherein X is an alcohol selected from the following: a) a sugar alcohol selected from _erythritol, pentaerythritol, xylitol, arabitol, ribitol, or mixtures thereof, b) a glycol comprising between 2 and 5 repeating subunits, wherein the repeating subunits are selected from an ethylene glycol, a 1,2-propylene glycol, a 1,3-propylene glycol, or mixtures thereof, c) a triol selected from glycerol, butanetriol, pentantriol, esantriol or mixtures thereof, or d) mixtures thereof, wherein X has two terminal oxygen atoms, wherein at least one of the oxygen atoms is configured to engage with at least one of the ester bonds, and wherein the rosin ester comprises rosin diesters and at least one second material, wherein the second material comprises at least one component, wherein, if: i) the second material comprises only one component, then the concentration in the slurry of rosin diesters is higher than said one component, or the second material comprises two or more components, then the concentration in the slurry of rosin diesters is higher than any single component of the second material, wherein the terpene polymer resin is an alpha-pinene polymer having between 3 and 5 α-pinene monomers.

The particles are as described above. The resin is as described above. The benefit agents are as described above. The fragrance material is as described above.

In a preferred aspect, the resin may have a softening point of less than 75°C, preferably of less than 50°C, more preferably of 45°C or less, most preferably of between 35°C and 45°C, and the alcohol may be selected from diethylene glycol, triethylene glycol, or mixtures thereof, preferably diethylene glycol, and at least one plant rosin acid may be an abietic acid.

In a preferred aspect, the rosin diesters may constitute at least 45%, preferably at least 70%, more preferably at least 75%, by weight of the rosin ester, preferably the weight ratio between the rosin diesters and at least one second material may be 9:1 to 1:1.5, preferably 8:1 to 1:1, more preferably 4:1 to 2:1, most preferably 3:1, more preferably whereinthe at least one second material may be selected from rosin monoesters, the at least one plant rosin acid, the alcohol or mixtures thereof.

### Method of treating fabrics

Another aspect of the present invention relates to a method of treating fabrics, the method comprising the following steps: A) obtaining a fabric to be washed; and B) contacting the fabric with a wash liquor, the wash liquor comprising the treatment composition according to the first aspect and water.

The process of treating fabrics comprises the step of obtaining a fabric to be treated, contacting the fabric with a wash liquor, comprising the treatment composition and water.

The treatment composition may be diluted in water prior, during, or after contacting the surface or article. The fabric may be optionally washed and/or rinsed before and/or after the contacting step. The composition may be applied directly onto a fabric or provided to a dispensing vessel or drum of an automatic laundry machine.

The method of treating a fabric may include the steps of: (a) optionally washing, rinsing and/or drying the fabric; (b) contacting the fabric with the treatment composition as described herein, optionally in the presence of water; (c) optionally washing and/or rinsing the fabric; (d) optionally drying, whether passively and/or via an active method such as a laundry dryer, or (e) combinations thereof. The method may occur during the wash cycle or the rinse cycle, preferably the rinse cycle, of an automatic washing machine. The fabric may be treated by a wash cycle and then followed by one or more rinse cycles.

For purposes of the present disclosure, treatment may include but is not limited to, scrubbing and/or mechanical agitation. The fabric may comprise most any fabric capable of being laundered or treated in normal consumer use conditions.

Wash liquors that comprise the disclosed compositions may have a pH of from about 3 to about 11.5. When diluted, such treatment compositions are typically employed at concentrations of from about 500 ppm to about 15,000 ppm in solution. When the wash solvent is water, the water temperature typically ranges from about 5 °C to about 90 °C and, the water to fabric ratio may be typically from about 1:1 to about 30:1.

The fabric may be contacted with an anionic surfactant, optionally in the presence of water, prior to being contacted with the treatment composition. The fabric may comprise anionic surfactant that is residual from a washing step. The source of the anionic surfactant may be a detergent composition, such as a heavy-duty liquid laundry detergent, a water-soluble pouch comprising a detergent composition, or a powdered laundry detergent. The detergent composition may further comprise suitable detergent adjuncts. For example, the detergent composition may further comprise cellulase enzyme, fatty acids and/or salts thereof, or mixtures thereof.

The treatment composition may be diluted with water, optionally in a vessel such as an automatic washing machine, to make a rinse liquor. The rinse liquor may comprise anionic surfactant and/or cellulase enzymes, which may be residual or carried over from a wash cycle. The rinse liquor may be removed from the vessel. The fabric may be dried by any suitable process, such as in an automatic dryer, or by line drying.

The water that is part of the wash liquor and/or the rinse liquor may be characterized by a certain hardness. For example, the water may be characterized by having less than 12 gpg, or less than 10 gpg, of hardness. It is believed that lower levels of hardness can lead to better performance compared to processes that occur in higher levels of hardness.

### Method of cleaning

In another aspect, the invention may relate to a method of cleaning fabrics, the method comprising the following steps:
i) obtaining a first fabric to be cleaned, wherein a resin according to the present invention is present on the first fabric; and
ii) contacting the first fabric with a first wash liquor, the first wash liquor comprising a first detergent composition and water,

The method of cleaning fabrics may further comprise one or both of the following steps:
i) contacting a second fabric with a second wash liquor, the second wash liquor comprising a treatment composition according to the present invention and water, wherein step iii occurs before step i of the present method.
ii) contacting a second fabric with a third wash liquor, the third wash liquor comprising a second detergent composition and water, wherein step iv occurs before step i or step iii of the present method.

It is believed that fabrics treated with treatment composition according to the present invention are more easily cleanable from deposited resin by appropriate detergents, as described above.

The resin on the fabric may be the resins as described above.

The second wash liquor may be obtained by the description described above for the method of treating fabrics.

The skilled person is aware of the composition of suitable first and second detergent compositions. Suitable first and second detergent compositions may comprise surfactants systems as described above.

The first and third wash liquor may be obtained by the non-limiting example described below:
The process of cleaning fabrics comprises the step of obtaining a fabric to be cleaned, contacting the fabric with a wash liquor, comprising detergent composition and water. Typically, the wash liquor has a temperature of above 0 °C to 90°C, or to 60 °C, or to 40°C, or to 30 °C, or to 20°C. The fabric may be contacted to the water prior to, or after, or simultaneous with, contacting the detergent composition with water. Typically, the wash liquor is formed by contacting the detergent composition to water in such an amount so that the concentration of detergent composition in the wash liquor is from 0.2g/l to 20g/l, or from 0.5g/l to 10g/l, or to 5.0g/l. The method of cleaning fabrics can be carried out in a front-loading automatic washing machine, top loading automatic washing machines, including high efficiency automatic washing machines, or suitable hand-wash vessels. Typically, the wash liquor comprises 90 litres or less, or 60 litres or less, or 15 litres or less, or 10 litres or less of water. Typically, 200g or less, or 150g or less, or 100g or less, or 50g or less of detergent composition is contacted to water to form the wash liquor.

### Combinations:

Clause 1: A treatment composition comprising particles and an adjunct ingredient, wherein the particles comprise a resin and one or more benefit agents, wherein the one or more benefit agents comprises a fragrance material, characterized in that the resin is selected from a rosin ester, a terpene polymer resin, or mixtures thereof, wherein the rosin ester has the following formula: wherein R1 and R2 are plant rosin acids or H, wherein at least one of R1 or R2 is a plant rosin acid, wherein E1 and E2 are covalent bonds, wherein at least one of E1 or E2 is an ester bond, wherein X is an alcohol selected from the following: a) a linear sugar alcohol selected from erythritol, xylitol, arabitol, ribitol, or mixtures thereof, b) a glycol comprising between 2 and 5 repeating subunits, wherein the repeating subunits are selected from an ethylene glycol, a 1,2-propylene glycol, a 1,3-propylene glycol, or mixtures thereof, c) a triol selected from glycerol, butanetriol, pentantriol, esantriol or mixtures thereof, or d) mixtures thereof, wherein X has two terminal oxygen atoms, wherein at least one of the oxygen atoms is configured to engage with at least one of the ester bonds, and wherein the rosin ester comprises rosin diesters and at least one second material, wherein the concentration of rosin diesters in the rosin ester is higher than the concentration of the at least one second material in the rosin ester, wherein the terpene polymer resin is an alpha-pinene polymer.

Clause 2. The composition according to clause 1, wherein the resin has a softening point of less than 75°C, preferably of less than 50°C, more preferably of less than 45°C, most preferably of between 35°C and 45°C.

Clause 3. The composition according to clauses 1 and 2, wherein the resin is characterized by at least one of the two following characteristics: a) an acid value between more than 0 and 20, preferably between 13 and 20, or even between 13 and 15, b) a color grade between 1 and 10, preferably between 1 and 7, more preferably between 2 and 6 as graded on the Gardner Color standard number.

Clause 4. The composition according to any preceding clause, wherein the alcohol is selected from diethylene glycol, triethylene glycol, or mixtures thereof, preferably diethylene glycol.

Clause 5. The composition according to any preceding clause, wherein at least one plant rosin acid is an abietic acid, and wherein at least one plant rosin acid is selected from a gum plant rosin acid, a wood plant rosin acid, a tall oil plant rosin acid, preferably a tall oil plant rosin acid.

Clause 6. The composition according to any preceding clause, wherein the rosin diesters constitute at least 45%, preferably at least 70%, more preferably at least 75%, by weight of the rosin ester.

Clause 7. The composition according to any preceding clause, wherein the weight ratio between the rosin diesters and at least one second material is 9:1 to 1:1.5, preferably 8:1 to 1:1, more preferably 4:1 to 2:1, most preferably 3:1, wherein the at least one second material is selected from rosin monoesters, the at least one plant rosin acid, the alcohol or mixtures thereof.

Clause 8. The composition according to any preceding clause, wherein the particles are characterized by a volume-weighted median particle size between 0.5 um and 40 um, preferably between 1 um and 20 um, more preferably between 1 um and 10 um.

Clause 9. A composition according to any preceding clause, wherein the weight ratio between the resin and the fragrance material in the composition is 1:4 to 4:1, preferably 1:4 to 2:1, more preferably 1:3 to 1.5:1, most preferably 1:1.

Clause 10. A composition according to any preceding clause, wherein the fragrance material comprises one or more perfume raw material, wherein the one or more perfume raw material comprises a moiety selected from the group consisting of a cycloalkane moiety, a cycloalkene moiety, a branched alkyl moiety, and combinations thereof, wherein the cycloalkane moiety comprises a first ring structure having from five to seven carbons, preferably from five to six carbons, that form the first ring structure, wherein the cycloalkene moiety comprises a second ring structure having from five to seven carbons, preferably from five to six carbons, that form the second ring structure, wherein the branched alkyl moiety comprises from one to four carbon atoms, preferably from two to three carbon atoms.

Clause 11. A composition according to any preceding clause, wherein the adjunct ingredient is selected from an amine, a surfactant system, a water binding agent, a sulfite, fatty acids and/or salts thereof, enzymes, encapsulated benefit agents, soil release polymers, hueing agents, builders, chelating agents, dye transfer inhibiting agents, dispersants, enzyme stabilizers, catalytic materials, bleaching agents, bleach catalysts, bleach activators, polymeric dispersing agents, soil removal/antiredeposition agents, polymeric dispersing agents, polymeric grease cleaning agents, brighteners, suds suppressors, dyes, hueing agents, a perfume delivery system, structure elasticizing agents, fabric softening agents, carriers, fillers, hydrotropes, organic solvents, anti-microbial agents and/or preservatives, neutralizers and/or pH adjusting agents, processing aids, fillers, rheology modifiers or structurants, opacifiers, pearlescent agents, pigments, anti-corrosion and/or anti-tarnishing agents, or mixtures thereof, preferably wherein the adjunct ingredient comprises a surfactant system, fabric softening agents, or combinations thereof, more preferably wherein the surfactant system comprises anionic surfactant, nonionic surfactant, cationic surfactant, and/or zwitterionic surfactant, and/or more preferably wherein the fabric softening agents comprise a quaternary ammonium compound, silicone compounds, or both, most preferably the adjunct ingredient is a quaternary ammonium compound.

Clause 12. A composition according to any preceding clause, wherein the composition may be liquid treatment composition or a solid treatment composition, preferably a liquid treatment composition, more preferably, wherein the composition is a liquid fabric care treatment composition, liquid hard surface treatment composition, liquid personal care treatment composition, most preferably a liquid fabric care treatment composition.

Clause 13. A consumer product comprising a bottle, wherein the treatment composition according to clauses 1-12 is contained in the bottle.

Clause 14. A method of making the treatment composition according to clauses 1-12, said process comprising the following steps: A) mixing a resin and one or more benefit agents to create an encapsulated perfume slurry; and B) using the slurry to produce the treatment composition.

Clause 15. The method of clause 14, wherein the slurry is mixed with a cationic surfactant to produce the treatment composition, preferably, wherein the cationic surfactant is a quaternary ammonium compound or a salt thereof.

Clause 16. A method of treating fabrics, the method comprising the following steps: a) obtaining a fabric to be washed; and b) contacting the fabric with a wash liquor, the wash liquor comprising the treatment composition according to clauses 1-12 and water.

Clause 17. An encapsulated perfume slurry comprising particles, wherein the particles comprise a resin and one or more benefit agents, wherein the one or more benefit agents comprises a fragrance material, characterized in that the resin is selected from a rosin ester, a terpene polymer resin, or mixtures thereof, wherein the rosin ester has the following formula: wherein R1 and R2 are plant rosin acids or H, wherein at least one of R1 or R2 is a plant rosin acid, wherein E1 and E2 are covalent bonds, wherein at least one of E1 or E2 is an ester bond, wherein X is an alcohol selected from the following: a) a linear sugar alcohol selected from erythritol, xylitol, arabitol, ribitol, or mixtures thereof, b) a glycol comprising between 2 and 5 repeating subunits, wherein the repeating subunits are selected from an ethylene glycol, a 1,2-propylene glycol, a 1,3-propylene glycol, or mixtures thereof, c) a triol selected from glycerol, butanetriol, pentantriol, esantriol or mixtures thereof, or d) mixtures thereof, wherein X has two terminal oxygen atoms, wherein at least one of the oxygen atoms is configured to engage with at least one of the ester bonds, and wherein the rosin ester comprises rosin diesters and at least one second material, wherein the concentration of rosin diesters in the rosin ester is higher than the concentration of the at least one second material in the rosin ester, wherein the terpene polymer resin is an alpha-pinene polymer.

Clause 18. A slurry of clause 17, wherein the resin has a softening point of less than 75°C, preferably of less than 50°C, more preferably of 45°C or less, most preferably of between 35°C and 45°C, and wherein the alcohol is selected from diethylene glycol, triethylene glycol, or mixtures thereof, preferably diethylene glycol, and wherein at least one plant rosin acid is an abietic acid.

Clause 19. A slurry of clauses 17-18, wherein the rosin diesters constitute at least 45%, preferably at least 70%, more preferably at least 75%, by weight of the rosin ester, and wherein the weight ratio between the rosin diesters and at least one second material is 9:1 to 1:1.5, preferably 8:1 to 1:1, more preferably 4:1 to 2:1, most preferably 3:1, and wherein the at least one second material is selected from rosin monoesters, the at least one plant rosin acid, the alcohol or mixtures thereof.

### Test Methods

The skilled person is deemed capable of performing the test methods for determining the parameters used to describe the present invention. Non-limiting examples of said test method methods are described below:

### Test Method for Determining the Logarithm of the Octanol/Water Partition Coefficient (logP)

The value of the log of the Octanol/Water Partition Coefficient (logP) is computed for each PRM in the perfume mixture being tested. The logP of an individual PRM is calculated using the Consensus logP Computational Model, version 14.02 (Linux) available from Advanced Chemistry Development Inc. (ACD/Labs) (Toronto, Canada) to provide the unitless 10 logP value. The ACD/Labs' Consensus logP Computational Model is part of the ACD/Labs model suite.

### Softening Point Test Method

If available, the softening point of a resin as provided by the manufacturer/supplier is to be used. If not available from the manufacturer/supplier, the softening point is determined according to ASTM E28-18, "Standard Test Methods for Softening Point of Resins Derived from Pine Chemicals," using the version approved July 1, 2018, and published July 2018. More specifically, the Reference Method ("Automated Ring and Ball Softening Point Method") provided therein is to be followed. The method is summarized here. As used herein (and as described in ASTM E28-18), the softening point is defined as the temperature at which a disk of the sample held with a horizontal ring (brass shouldered ring; 19.8 mm inner ring diameter, 23.0 outer diameter, as indicated in the ASTM method) is forced downward a distance of 25.4 mm (1 in.) under the weight of a steel ball (9.53mm diameter; mass between 3.45 and 3.55 g) as the sample is heated at 5C/min in a water, glycerin, silicone oil, ethylene glycol/water, or glycerin/water bath.

*Sample Preparation:* Select a representative sample of the resin to be tested. The sample should include flakes, pastilles, or freshly broken lumps free of oxidized surfaces; avoid inclusion of finely divided material or dust. Melt the sample in a clean bottle; avoid overheating and avoid incorporating air bubbles into the sample. The time from the beginning of heating to the pouring of the sample should not exceed 15 minutes. Rest the ring, bottom down, on a metal surface; the ring may be preheated. Pour the melted resin sample into the ring so as to leave an excess upon cooling. After cooling for at least 30 minutes, remove excess material from the periphery and top of the ring.

*Bath Liquid:* The selection of the bath liquid will depend on the softening point ("SP") of the resin. For SPs between 35C and 80C, use water (distilled or deionized, freshly boiled). For SPs between 80C and 150C, use USP Glycerin. For SPs above 80C, use Silicone Oil (Polydimethylsiloxane - 200 fluid, 50 cSt, from Dow Coming, Midland, MI). For SPs up to 35C, use a 50/50 (v/v) mixture of Ethylene Glycol and Distilled Water; the bath should be cooled 10 to -25C in a precooled freezer or an isopropyl dry-ice bath.

*Test:* Use a suitable automated ring and ball-softening point instrument with control unit; calibrate according to the manufacturer's instructions. Provide a stir bar to a 600 mL beaker and fill with a bath liquid as provided above, depending on the softening point of the resin. Set up the apparatus, ring, ball, test insert, support pins as recommended by the manufacturer's instructions. Verify that the control unit is set for the correct bath liquid. Heat the bath so that the temperature of the bath liquid is raised uniformly at a rate of 5C/min. The test is complete when then light beam has been interrupted by the falling ball and material. Record the softening point at the temperature displayed on the unit after the test is completed.

### Acid Number Test Method

If available, the acid number of a resin as provided by the manufacturer/supplier is to be used. If not available from the manufacturer/supplier, the acid value is determined according to ASTM D465-15 (Reapproved 2020), "Standard Test Methods for Acid Number of Pine Chemical Products Including Tall Oil and Other Related Products," as approved June 1, 2020 and published June, 2020. More specifically, the Referee Method ("Potentiometric Method") provided therein is to be followed. The method is summarized here. Provide freshly chipped samples of resin, which may be further crushed to facilitate weighing and dissolution; pieces with oxidized surfaces, as well as existing resin dust or powder, should not be used. If a nonhomogenous liquid, place in a closed bottle with a capillary vent or its equivalent, and heat in a hot water bath; the sample may be agitated during heat, and used after homogenous and well stirred. Based on the following table, transfer the proscribed amount of sample to a 400 mL tall form beaker; add the proper amount of solvent I and swirl to dissolve, heating gently if necessary. Add the proper amount of solvent II, if required, and cool to near room temperature. Immerse each electrode of a glass electrode pH meter (calibrated/standardized according to the manufacturer's instructions) in the solution. Stir with a stir bar. Titrate with a standard alkali solution (a 0.5 N or 0.1 N KOH solution), recording the buret and pH meter readings. Sufficient alkali may be added to bring the pH of the solution to 8. Add alkali in 1.0 mL portions until the change in pH per increment added amounts to 0.3 pH unit. Reduct the additions of alkali to 0.1 mL or smaller until the end point has been passed, as indicated by a significant decrease in pH units er 0.1 mL added. Continue the titration with 1.0 mL portions until it becomes apparent that the inflection point has been well defined. Determine the inflection point (point of maximum change in pH per mL of alkali solution) to the nearest 0.05 mL by plotting the pH readings against the milliliters of alkali used.
(For more accuracy, the chance in pH per mL may be plotted against the pH; the peak corresponds to the inflection point.) The inflection point is considered the end point of the titration.

The acid number of the sample, expressed as milligrams of KOH per gram of sample is calculated as follows, and may be reported to the nearest whole number:$Acid Number = \left(A x N x 56.1\right) / B$ where: A = alkali solution (in mL) required for titration of the specimen; N = normality of the alkali solution, and B = specimen weight (in grams).

### Color Grade Test Method (Gardner Color)

If available, the color grade (Gardner color) of a resin as provided by the manufacturer/supplier is to be used. If not available from the manufacturer/supplier, the color grade (Gardner color) is determined according to ASTM D6166-12 (Reapproved 2016), "Standard Test Method for Color of Pine Chemicals and Related Products (Instrumental Determination of Gardner Color)," as approved December 1, 2016, and published December, 2016. The method is summarized here. The color of a liquid sample is measured using an instrument, such as a Gardner Color Comparator L, 115V (ex. BYK), capable of measuring transmitted color and reporting in Gardner colors (or, less preferred, in a color system that can be converted to Gardner colors by known methods, such as those disclosed in the ASTM D6166-12). The instrument is calibrated according to the manufacturer's instructions. To prepare the resin sample for color analysis, a molten sample of the resin is introduced to a glass cuvet (10-mm path, unless a different path length is specified by the instrument manufacturer). If the sample is solid, it should comprise freshly broken lumps and be free of dust and finely divided material; the solid should be melted (e.g. in 15 minutes or less, in an oven, sand bath, or oil bath), taking care to avoid overheating and introduction of bubbles. After the molten sample is introduced to the glass cuvet, measurements should be taken while still molten. If the material is cloudy, it should be filtered. The glass cuvet is inserted into the instrument, and the color is measured by following the manufacturer's instructions.

### Flash Point Test Method

If available, the flash point of a resin as provided by the manufacturer/supplier is to be used. If not available from the manufacturer/supplier, the flash point is determined according to ASTM D92-18, "Standard Test Methods for Flash and Fire Points by Cleveland Open Cup Tester," as approved July 1, 2018, and published July, 2018.

### Fabric Treatment Method

When treating fabrics with a composition according to the present disclosure in the experiments below, the following method is followed unless otherwise indicated. For each treatment, a washing machine (ex Miele) is loaded with 3kg of a fabric load. The fabric load comprises 1065g knitted cotton fabric and 1065g polyester-cotton fabrics (50/50). Additionally, the fabric load comprises twenty terry towel tracers, which weigh together 870g. The washing cycle is run at 95°C.

Prior to the test treatment, the load is preconditioned twice, each time using the 95°C short cotton cycle with 79g of unperfumed IEC A Base detergent (ex WFK Testgewebe GmbH), followed by two additional 95°C washes without detergent. For the test treatment, the load is washed using a 40°C short cotton cycle, 1200rpm spin speed with 79g IEC A Base detergent, which is added at the start of the wash cycle in the appropriate dispenser. A dosage of 40 ml of the test fabric treatment composition is added in the appropriate dispenser.

### Viscosity Method

Viscosity of a liquid composition is measured using a DV-E viscometer from Brookfield. The spindle is automatically spun at a rate of 60 rpm until a stable value is given in centipoise (cP). Viscosity of the slurry comprising resin, delivery agent and potentially emulsifying agent is measured using a HAAKE MARS from Thermo Scientific using a 60 mm 1° Cone and a gap size of 52 micrometers. The shear viscosity at 20s-1 can be obtained from a logarithmic shear rate sweep from 0.01s-1 to 1200s-1 at 21°C. The viscosity may be expressed as centipoise (cP).

### Particle Size Determination

The volume-weighted median particle size of the particles is calculated from the values obtained by microscopically observing and measuring the diameter of around 900 capsules observed in randomly sampled aliquots. Images of the concentrated slurries and compositions were collected using an inverted DIAPOT 300 microscope (Nikon, Tokyo) connected to a CCD camera with a digital recorder (Nikon digital sight DS-U1). Few microliters of the solutions were placed on a microscope slide, covered with a glass slip and observed in transmitted light. The images were collected using a 20x objective.

The outputs obtained after the microscopy analysis are: (1) list of diameters detected; and (2) counts per each diameter size detected.

Therefore, the volume (V) of each particle is calculated with the following equation: $V = \frac{4}{3} π {r}^{3}$ where r is the radius of each detected particle. Finally, the volume-weighted median particle size is calculated (e.g., via a spreadsheet, such those created in Microsoft ExcelTM), assuming that each particle is a sphere.

### Confocal Raman microscopy on liquid formulations

Confocal Raman microscopy was carried out using a Renishaw InViaTM Qontor confocal microRaman system equipped with a front illuminated CCD camera and a research-grade Leica DM 2700 microscope. Maps were collected using a 50x objective (WD 8.2 mm, NA 0.50) and a laser operating at 532 nm with a power of 50 mW and a grating of 1800 l/mm. For each sample, several maps were collected in different regions of the surface. The exposure time for each spectrum was 1 s, in the range 200-1900 cm-1 and a step of 2 µm. Spectra were processed with the Renishaw software WiRETM, corrected for cosmic rays, baseline and noise, and then used to obtain maps. The spectra of the pure phases constituting the samples (perfume raw materials, resins, treatment composition, cotton and polyester) were also collected. All spectra were collected using a 50x objective, 100 mW, 1 s per spectrum, and 10 accumulations, and used as reference spectra to perform the analysis of the maps with the WiRETM software.

### Confocal Raman microscopy on model fabrics

For the evaluation of the presence of microcapsules on cotton and PE, fabrics were investigated through 2D confocal Raman microscopy, using a Renishaw InViaTM Qontor confocal microRaman system equipped with a front illuminated CCD camera and a research-grade Leica DM 2700 microscope. Maps were collected using a 50x objective (WD 8.2 mm, NA 0.50) and a laser operating at 532 nm with a power of 100 mW and a grating of 1800 l/mm. For each sample, several maps were collected in different regions of the surface. The exposure time for each spectrum was 1 s, with 1 accumulation, in the range 200-1900 cm-1 (10% power, step size 5 µm). Spectra were processed with the Renishaw software WiRETM, corrected for cosmic rays, baseline and noise, and then used to obtain maps.

### Examples

The examples provided below are intended to be illustrative in nature and are not intended to be limiting.

### Example 1.

Table 1 shows a variety of commercially available resins. Additional information is provided where available.

**Table 1. Commercially available resins**

| **No.** | **Resin Type** | **Derivative Type** | **Softening Point (°C)** | **Acid Value (mg KOH / g)** | **GARDNER COLOR 50R/50 TOL** | **TRADE NAME** | **Supplier** |
|---|---|---|---|---|---|---|---|
| 1 | Tall oil | di-ethylene glycol ester | 35-45 | 13-20 | 2.5 | Dertoline DEG2 | DRT |
| 2 | Wood or Gum | Tri-ethylene glycol ester of rosin | liquid | < 15 | 6 | Granolite TEG | DRT |
| 3 | Wood | Tri-ethylene glycol ester of partially hydrogenated rosin | 55-72 | < 15 | 6 | Staybelite Ester3 | PINOVA |
| 4 | Resin | Polyterpenoid resin | liquid | < 1 | < 7 | Dercolyte LTG | DRT |

### Example 2

Liquid fabric softener compositions according to Table 2, below, are provided.

**Table 2. Liquid fabric softener compositions**

| Total composition | A | Comparative composition B | Comparative composition C |
|---|---|---|---|
| Ingredient name | WT% | | |
| water | 90.898 | 91.688 | 89.888 |
| HEDP monosodium salt solution | 0.030 | 0.030 | 0.030 |
| Formic Acid 85% | 0.045 | 0.045 | 0.045 |
| HCl 32% | 0.020 | 0.020 | 0.020 |
| Quaternary ammonium compound (ester quat) | 5.82 | 5.03 | 5.03 |
| Antifoam Emulsion | 0.0170 | 0.0170 | 0.0170 |
| Structurant | 0.17 | 0.17 | 0.17 |
| Non-ionic surfactant (BT7 from Nikkol) | 0 | 0 | 1.8 |
| Permalyn 5095 | 0 | 1.50 | 1.50 |
| Dertoline DEG2 | 1.50 | 0 | 0 |
| Perfume Raw Material | 1.50 | 1.50 | 1.5 |

According to table 2, compositions A-C share a formulation base but have the following differences:
Composition A comprises Dertoline DEG2, a resin according to the present invention. Dertoline DEG2 comprises a mixture of free rosin acids, rosin monoesters, and rosin disters, among which rosin disters are the most present component.

The comparative compositions B and D (hereafter called B and D) comprise Permalyn 5095, a resin outside of the scope of the present invention. Composition D also comprises an emulsifying agent (i.e. a non ionic surfactant).

Compositions B and D do not fall within the scope of the invention and they are merely used as comparative compositions.

### Example 3

Compositions A-C were tested to determine the stickiness of the compositions towards the walls of containers made of different materials. The compositions were stored either in transparent bottles made of PET (Polyethylene Terephthalate) or in transparent glass bottles.

The test in glass bottles was performed as follows. 20 grams of the composition were prepared in 40 mL-glass bottles, sealed, and stored in upright position for two months.

The test in PET bottles was performed as follows: 100 grams of the composition were prepared in 250 mL-PET bottles, sealed, and stored in upright position for three weeks.

After storage, the bottles were inverted and/or shaken. Pictures were collected for each sample (upright position, upside-down position, after manual shaking) and the visual assessment of stickiness was carried out as described above.

The stickiness of the compositions was determined by macroscopic observation of a) insoluble residues left on the walls of the container and b) oil-water phase separation phenomena.

Values relative to stickiness were assigned (see Table 3A) from the following scale:
1 - No insoluble residues, no phase separation
2 - No or few insoluble residues, phase separation present but easily reversible by shaking
3 - Several insoluble residues present, reversible phase separation by shaking
4 - Multiple residues present, irreversible phase separation by shaking

**Table 3A. Stickiness to the container's material**

| Container type | A | B | C |
|---|---|---|---|
| PET bottle | **1** | **4** | **1** |
| Glass bottle | **1** | **4** | **1** |

Composition A leaves no insoluble residues and show no water-oil phase separation, contrary to composition B. The resin according to the present invention allows an optimal stability of the composition. This result can be achieved by other types of resin only upon addition of a non-ionic surfactant (composition C).

The resin according to the present invention clearly provides a benefit in terms of reducing the visible residues left on the walls of the container without the need for an emulsifying agent. This is valid for both PET and glass containers and across different timepoints of storage.

Secondarily, compositions A-C were analyzed by optical microscopy to determine the stability of microcapsules. As microcapsules are expected to be stable during the washing cycle, the compositions were diluted 1:100 in water, mixed for 2 min and monitored with optical microscopy according to the test method described above.

The stability of the microcapsules was determined visually from the microscopy images based on the a) form of the particles and b) presence of residual elements, such as coalesced microcapsules.

Values relative to microcapsules stability were assigned (see Table 3B) from the following scale:
1 - Spherical microcapsules, no residual elements
2 - Semi- spherical microcapsules, few residual elements
3 - Non-spherical microcapsules, several residual elements
4 - Non-spherical microcapsules, abundant residual elements

**Table 3B. Microcapsules stability**

| A | B | C |
|---|---|---|
| **1** | **3** | **1** |

Compositions A shows abundant spherical microcapsules and no residual elements, whereas composition B shows unstable and non-spherical microcapsules. This proves that the use of a resin according to the present invention improves the stability of the microcapsules. Other types of resins need an emulsifying agent in order to achieve the same result (composition C).

### Example 4

Compositions A-C were tested to determine their processability and flowability on materials commonly found in manufacturing/transportation facilities, such as stainless steel and silicone.

The residue buildup of the compositions on stainless steel and silicone was assessed visually.

The residue buildup on stainless steel was assessed using a 8 x 2 cm2 stainless steel foil. The foil was submerged in 40 mL of the investigated composition, left shaking in a VWR standard analog orbital shaker (speed 5) for 24 hours, rinsed with abundant water and air dried.

After visual assessment of residue build-up left on the stainless steel material, values were assigned (see Table 4) from the following scale:
1 - no visible residue, build-up < 0.1%
2 - no visible residue, build-up 0.1 - 1%
3 - little residue, build-up 1-6 %
4 - visible residue, build up > 7%

The residue buildup on a silicone material was assessed using a microfluidics device having silicone channels. The original sample (not diluted) is loaded into a 1 ml syringe. The syringe is loaded on the Nemesys platform (Syringe pump). The syringe pump is set with a flow rate parameter of 0.01 µL/s. The syringe outlet is connected to the PDMS microfluidic chip. The selected channel on the microfluidic chip is analyzed using a confocal microscope. The skilled person is aware of the structure and functioning of commonly-used microfluidics devices.

After visual assessment of the residues left on the silicone material, values were assigned (see Table 4):
1 - flow is continuous, no lumps
2 - flow is hindered by lumps forming after 20 minutes of flowing
3 - flow is hindered by lumps forming after 5 minutes of flowing
4 - flow is blocked by lump within 1 minute of flowing

**Table 4. Processability/flowability of the compositions**

| Material | A | B | C |
|---|---|---|---|
| Stainless steel | **1** | **4** | **3** |
| Silicone | **1** | **3** | **1** |

Composition A shows no residue buildup in both stainless steel and silicone materials. Composition B show low processability in both materials which is rescued by the emulsifying agent partially on stainless steel and fully on silicone (composition C).

Overall, the use of a resin according to the present invention confers optimal processability and flowability to the composition across different materials without the need for an emulsifying agent.

These results highlight the potential for these resins to improve the processes of manufacturing, handling and the storage of treatment compositions.

### Example 5

Compositions A-C were tested to determine the efficiency of particle deposition on cotton fabrics.

The composition samples were diluted 1:10 in water. White pure cotton was used as model clothes. The fabrics were cut in 2 × 2 cm² pieces and submerged in a water solution with the samples. After 1 h of mixing at VWR^{®} standard analog orbital shaker (speed 6), the fabrics were removed, dried, placed on a glass slide and mapped by means of 2D confocal Raman microscopy.

Based on visual assessment of the signal from the confocal imaging, the level of particle deposition was assigned values (see Table 5) from the following scale:
1 - Abundant
2 - Present
3 - Scarce
4 - Absent

**Table 5. Deposition on cotton fabrics**

| A | B | C |
|---|---|---|
| 1 | 1 | 1 |

Compositions A-C show comparable deposition efficiencies on cotton fibers. Overall, the use of resins according to the present invention does not alter the particle deposition efficiency of the composition.

### Example 6

Compositions A-C were tested to determine the degree of removal of deposited resin on the cotton fabrics.

The fabrics were treated with compositions according to the procedure described in Example 5. Fabrics having comparable staining pattern were washed by shaking them at VWR standard analog orbital shaker (speed 6) in a water solution with 5% v/v abundant laundry detergent (liquid Dash detergent) for 60 minutes, rinsed with abundant water and dried. Then, the fabrics were mapped through 2D confocal Raman microscopy, to check for the presence of resin residues.

Based on visual assessment of the signal from the confocal imaging, values were assigned (see Table 6) from the following scale:
1 - Complete
2 - Almost complete
3 - Partial
4 - Null

**Table 6. Removal of deposited resin**

| A | B | C |
|---|---|---|
| **1** | **3** | **1** |

Deposited resin on cotton fabric was completely removed when fabrics were treated with composition A, but not with composition B. The use of resins according to the present invention makes the deposited resin more prone to be removed when the fabric is washed with an appropriate detergent, similarly to other types of resins formulated with an emulsifying agent (composition C).

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "40 mm."

## Claims

1. A treatment composition comprising particles and an adjunct ingredient,
wherein the particles comprise a resin and one or more benefit agents,
wherein the one or more benefit agents comprises a fragrance material, **characterized in that** the resin is selected from a rosin ester, a terpene polymer resin, or mixtures thereof,
wherein the rosin ester has the following formula: 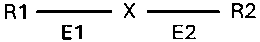
wherein R1 and R2 are plant rosin acids or H, wherein at least one of R1 or R2 is a plant rosin acid,
wherein E1 and E2 are covalent bonds, wherein at least one of E1 or E2 is an ester bond,
wherein X is an alcohol selected from the following:
a) a sugar alcohol selected from erythritol, pentaerythritol, xylitol, arabitol, ribitol, or mixtures thereof,
b) a glycol comprising between 2 and 5 repeating subunits, wherein the repeating subunits are selected from an ethylene glycol, a 1,2-propylene glycol, a 1,3-propylene glycol, or mixtures thereof,
c) a triol selected from glycerol, butanetriol, pentantriol, esantriol or mixtures thereof,
or
d) mixtures thereof,
wherein X has two terminal oxygen atoms, wherein at least one of the oxygen atoms is configured to engage with at least one of the ester bonds,
and
wherein the rosin ester comprises rosin diesters and at least one second material,
wherein the second material comprises at least one component,
wherein, if:
i) the second material comprises only one component, then the concentration in the composition of rosin diesters is higher than said one component,
or
ii) the second material comprises two or more components, then the concentration in the composition of rosin diesters is higher than any single component of the second material,
wherein the terpene polymer resin is an alpha-pinene polymer having between 3 and 5 α-pinene monomers.

2. The composition according to claim 1, wherein the resin has a softening point of less than 75°C, preferably of less than 50°C, more preferably of less than 45°C, most preferably of between 35°C and 45°C.

3. The composition according to claims 1 and 2, wherein the resin is **characterized by** at least one of the two following characteristics:
a) an acid value between more than 0 and 20, preferably between 13 and 20, or even between 13 and 15,
b) a color grade between 1 and 10, preferably between 1 and 7, more preferably between 2 and 6 as graded on the Gardner Color standard number.

4. The composition according to any preceding claim, wherein the alcohol is selected from diethylene glycol, triethylene glycol, or mixtures thereof, preferably diethylene glycol.

5. The composition according to any preceding claim, wherein at least one plant rosin acid is an abietic acid, and wherein at least one plant rosin acid is selected from a gum plant rosin acid, a wood plant rosin acid, a tall oil plant rosin acid, preferably a tall oil plant rosin acid.

6. The composition according to any preceding claim, wherein the rosin diesters constitute at least 45%, preferably at least 70%, more preferably at least 75%, by weight of the rosin ester.

7. The composition according to any preceding claim, wherein the weight ratio between the rosin diesters and at least one second material is 9:1 to 1:1.5, preferably 8:1 to 1:1, more preferably 4:1 to 2:1, most preferably 3:1,
preferably, wherein the at least one component is selected from a rosin monoester, the at least one plant rosin acid, the alcohol or mixtures thereof.

8. The composition according to any preceding claim, wherein the particles are **characterized by** a volume-weighted median particle size between 0.5 um and 40 um, preferably between 1 um and 20 um, more preferably between 1 um and 10 um, wherein the volume-weighted median particle size is calculated according to the method described herein.

9. A composition according to any preceding claim, wherein the weight ratio between the resin and the fragrance material in the composition is 1:4 to 4:1, preferably 1:4 to 2:1, more preferably 1:3 to 1.5:1, most preferably 1:1.

10. A composition according to any preceding claim, wherein the fragrance material comprises one or more perfume raw material, wherein the one or more perfume raw material comprises a moiety selected from the group consisting of a cycloalkane moiety, a cycloalkene moiety, a branched alkyl moiety, and combinations thereof,
wherein the cycloalkane moiety comprises a first ring structure having from five to seven carbons, preferably from five to six carbons, that form the first ring structure,
wherein the cycloalkene moiety comprises a second ring structure having from five to seven carbons, preferably from five to six carbons, that form the second ring structure,
wherein the branched alkyl moiety comprises from one to four carbon atoms, preferably from two to three carbon atoms.

11. A composition according to any preceding claim, wherein the adjunct ingredient is selected from an amine, a surfactant system, a water binding agent, a sulfite, fatty acids and/or salts thereof, enzymes, encapsulated benefit agents, soil release polymers, hueing agents, builders, chelating agents, dye transfer inhibiting agents, dispersants, enzyme stabilizers, catalytic materials, bleaching agents, bleach catalysts, bleach activators, polymeric dispersing agents, soil removal/antiredeposition agents, polymeric dispersing agents, polymeric grease cleaning agents, brighteners, suds suppressors, dyes, hueing agents, a perfume delivery system, structure elasticizing agents, fabric softening agents, carriers, fillers, hydrotropes, organic solvents, anti-microbial agents and/or preservatives, neutralizers and/or pH adjusting agents, processing aids, fillers, rheology modifiers or structurants, opacifiers, pearlescent agents, pigments, anti-corrosion and/or anti-tarnishing agents, or mixtures thereof,
preferably wherein the adjunct ingredient comprises a surfactant system, fabric softening agents, or combinations thereof,
more preferably wherein the surfactant system comprises anionic surfactant, nonionic surfactant, cationic surfactant, and/or zwitterionic surfactant, and/or more preferably wherein the fabric softening agents comprise a quaternary ammonium compound, silicone compounds, or both,
most preferably the adjunct ingredient is a quaternary ammonium compound.

12. A composition according to claims 1-11, wherein the composition may be liquid treatment composition or a solid treatment composition, preferably a liquid treatment composition,
more preferably, wherein the composition is a liquid fabric care treatment composition, liquid hard surface treatment composition, liquid personal care treatment composition, most preferably a liquid fabric care treatment composition.

13. A method of making the treatment composition according to claims 1-12, said process comprising the following steps:
A) mixing a resin and one or more benefit agents to create an encapsulated perfume slurry; and
B) using the slurry to produce the treatment composition, preferably, wherein the slurry is mixed with a cationic surfactant to produce the treatment composition, and wherein the benefit agent comprises a fragrance material,
more preferably, wherein the cationic surfactant is a quaternary ammonium compound or a salt thereof.

14. A method of treating fabrics, the method comprising the following steps:
a) obtaining a fabric to be washed; and
b) contacting the fabric with a wash liquor, the wash liquor comprising the treatment composition according to claims 1-12 and water.

15. An encapsulated perfume slurry comprising particles,
wherein the particles comprise a resin and one or more benefit agents,
wherein the one or more benefit agents comprises a fragrance material,
**characterized in that** the resin is selected from a rosin ester, a terpene polymer resin, or mixtures thereof,
wherein the rosin ester has the following formula: 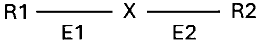
wherein R1 and R2 are plant rosin acids or H, wherein at least one of R1 or R2 is a plant rosin acid,
wherein E1 and E2 are covalent bonds, wherein at least one of E1 or E2 is an ester bond,
wherein X is an alcohol selected from the following:
a) a sugar alcohol selected from erythritol, pentaerythritol, xylitol, arabitol, ribitol, or mixtures thereof,
b) a glycol comprising between 2 and 5 repeating subunits, wherein the repeating subunits are selected from an ethylene glycol, a 1,2-propylene glycol, a 1,3-propylene glycol, or mixtures thereof,
c) a triol selected from glycerol, butanetriol, pentantriol, esantriol or mixtures thereof, or
d) mixtures thereof,
wherein X has two terminal oxygen atoms, wherein at least one of the oxygen atoms is configured to engage with at least one of the ester bonds,
and wherein the rosin ester comprises rosin diesters and at least one second material, wherein the second material comprises at least one component,
wherein, if:
i) the second material comprises only one component, then the concentration in the slurry of rosin diesters is higher than said one component,
or
ii) the second material comprises two or more components, then the concentration in the slurry of rosin diesters is higher than any single component of the second material,
wherein the terpene polymer resin is an alpha-pinene polymer having between 3 and 5 α-pinene monomers.

16. A slurry of claim 15, wherein the resin has a softening point of less than 75°C, preferably of less than 50°C, more preferably of 45°C or less, most preferably of between 35°C and 45°C,
preferably, wherein the alcohol is selected from diethylene glycol, triethylene glycol, or mixtures thereof, preferably diethylene glycol,
more preferably, wherein at least one plant rosin acid is an abietic acid.

17. A slurry of claims 15-16, wherein the rosin diesters constitute at least 45%, preferably at least 70%, more preferably at least 75%, by weight of the rosin ester,
preferably wherein the weight ratio between the rosin diesters and at least one second material is 9:1 to 1:1.5, preferably 8:1 to 1:1, more preferably 4:1 to 2:1, most preferably 3:1,
more preferably wherein the at least one component is selected from a rosin monoester, the at least one plant rosin acid, the alcohol or mixtures thereof.
